(19) Europäisches Patentamt
European Patent Office
Office européen des brevets

(11) EP 2 213 667 A1

(12) # EUROPEAN PATENT APPLICATION
published in accordance with Art. 153(4) EPC

(43) Date of publication:
**04.08.2010 Bulletin 2010/31**

(21) Application number: **07816841.6**

(22) Date of filing: **15.11.2007**

(51) Int Cl.:
***C07D 305/14*** (2006.01) ***A61K 31/337*** (2006.01)
***A61P 35/00*** (2006.01)

(86) International application number:
**PCT/CN2007/003235**

(87) International publication number:
**WO 2009/062342 (22.05.2009 Gazette 2009/21)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Institute of Mataria Medica, Chinese Academy of Medical Sciences**
**Beijing 100050 (CN)**

(72) Inventors:
- **FANG, Weishuo**
  **Beijing 100050 (CN)**
- **CHEN, Xiaoguang**
  **Beijing 100050 (CN)**
- **YANG, Chungang**
  **Beijing 100050 (CN)**
- **LI, Xuan**
  **Beijing 100050 (CN)**
- **WANG, Hongbo**
  **Beijing 100050 (CN)**
- **LIU, Hongyan**
  **Beijing 100050 (CN)**
- **HAN, Rui**
  **Beijing 100050 (CN)**
- **ZHAO, Limin**
  **Beijing 100050 (CN)**

(74) Representative: **Hart-Davis, Jason et al**
**Cabinet Beau de Loménie**
**158, rue de l'Université**
**75340 Paris Cedex 07 (FR)**

(54) **CEPHALOMANNINE DERIVATIVES AND THEIR PREPARATION, MEDICINAL COMPOSITION AND USE**

(57) The present invention discloses cephalomannine derivatives of general formula (I), a process for preparation of such cephalomannine derivatives, a composition containing such compounds, and use of said compounds in the manufacture of a medicament for the treatment of tumors, especially multidrug resistant tumors.

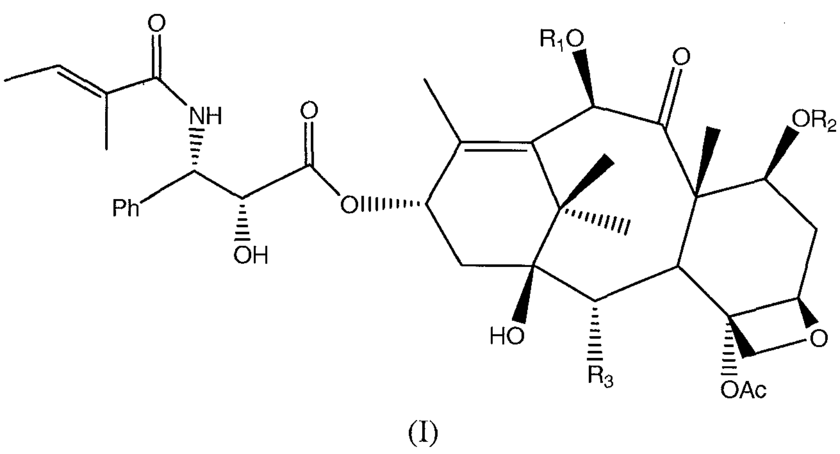

(I)

EP 2 213 667 A1

## Description

### Field of the Invention

The present invention discloses cephalomannine derivatives of general formula (I), a process for the preparation of such cephalomannine derivatives, a composition containing such compounds, and use of said compounds in the manufacture of a medicament for the treatment of tumors, especially multidrug resistant tumors.

### Background of the Invention

Taxanes belong to terpenes, and are of interest in both biological and chemical fields. Taxol (paclitaxel), one of taxanes, exhibits good inhibitory activity against tumors. It has been marketed as anticancer drug, and has the following structure:

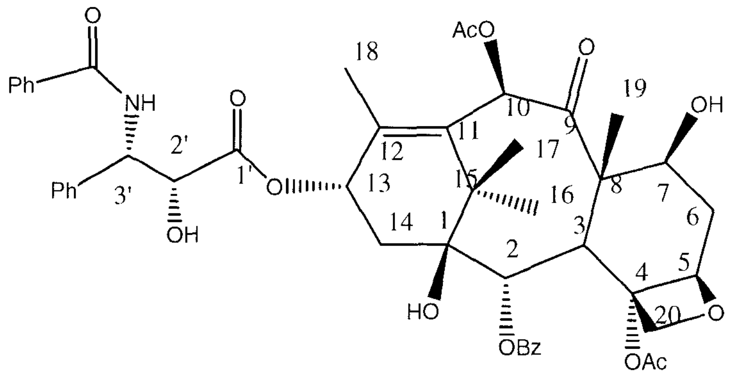

wherein, Ac is acetyl, and Bz is benzoyl.

U.S. Patent No. 4,814,470 to Colin *et. al.* has disclosed docetaxel, an analogue of taxol, the activity of which is significantly stronger than taxol. Docetaxel has also been marketed as anticancer drug, and has the following structure:

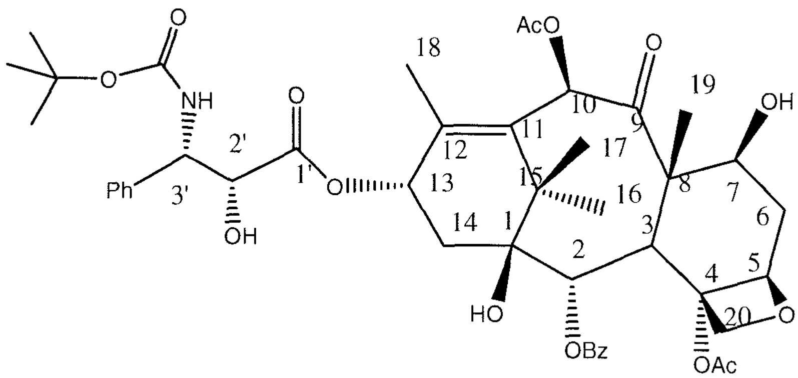

Taxol and docetaxel have a unique mechanism of action. They mainly promote the polymerization of tubulins and inhibit the depolymerization thereof, resulting in the abnormal arrangement of microtubule bundles and the formation of cladosclereids. Thus, the spindles lose normal functions, and the cell cycle is blocked in G2/M period, leading to the death of cancer cells (Schiff, P. B.; Fant, J.; Horwitz S. B. Nature. 1979, 277: 665). They are currently best-selling anticancer drugs, because of their unique and significantly useful effect against a variety of tumors which cannot be treated by other anticancer drugs.

Taxol has obtained great success in clinical therapy. However, it still has some disadvantages to be overcome. The inefficacy in chemotherapy of taxol against patients with primary and secondary drug-resistant tumors become a more and more serious problem in the use of taxol, beside other disadvantages e.g. poor water-solubility, limited resources, and the like. In addition, docetaxel is also ineffective on tumors which are resistant to taxol. These problems greatly restricted the application of taxol and docetaxel. The development of novel taxanes anticancer drugs effective on multidrug resistant tumors is of great importance.

It is well known that one of major factors rendering the failure of tumor chemotherapy is the multidrug resistance of tumors. The reasons for the occurrence of multidrug resistance are very complex. The drug resistance of taxol is mainly associated with over expression of P-glycoproteins and mutations of tubulins which are the targets of this kind of drugs. The drug resistance mediated by tubulins may arise from mutations of amino acid residues. In addition, the binding ability of taxol to variant tubulins decreases, resulting in the decrease of activity of taxol. P-glycoproteins can move a variety of ectogenic compounds, including chemotherapeutic drugs, out of the cells by active transport, rendering the concentration of the compounds in tumor cells less than the effective concentration.

The drug resistant tumors can be overcome mainly by the following routes: (1) administering the anticancer drugs in combination with MDR reversal agents, particularly P-glycoprotein inhibitors; (2) discovering novel anticancer drugs effective on drug resistant tumors. Researches based on the first route have been generally carried out, and they are theoretically possible, furthermore, some good experimental results both in vivo and in vitro have been reported. However, it is unfortunate that no clinically ideal effect has been obtained up to now. Therefore, the attention was turned toward the second route, i.e. developing new anticancer drugs which are also effective on drug resistant tumors. This has become a research focus of taxanes all over the world. Some second generation taxane compounds effective on multidrug resistant tumors, e.g. BMS-148876, IDN-5109, have been reported. The activity of these compounds against multidrug resistant tumors is significantly higher than taxol and docetaxel, and currently, they have been advanced into the clinical trial phase.

Cephalomannine is analogous to taxol in structure (show below), its content in plants is high (1 to 2 ‰, comparable to the content of taxol), and its effect on sensitive and resistant tumors is similar to taxol. But cephalomannine currently was not used sufficiently, because it usually was discarded as side product of taxol during industrial separation. Now, we made modification using cephalomannine, an analogue of taxol, as leading compound, this is beneficial for both the development of new drugs and the full utility of resources.

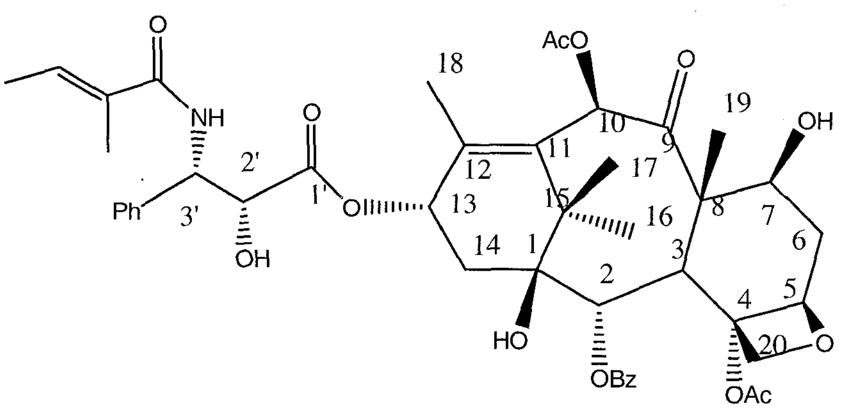

## Contents of the Invention

A technical problem to be solved by the present invention is to provide novel cephalomannine derivatives which are effective on the tumors resistant to taxol and docetaxel.

Another technical problem to be solved by the present invention is to provide a novel process for the preparation of said cephalomannine derivatives.

Still another technical problem to be solved by the present invention is to provide a pharmaceutical composition comprising a compound of general formula (I) as active ingredient and a pharmaceutically acceptable carrier.

Yet another technical problem to be solved by the present invention is to provide the use of novel cephalomannine derivatives and compositions containing them as antineoplastic agent.

To solve technical problems as mentioned above, the present invention provides the following technical solutions:

Specifically, cephalomannine derivatives of the present invention are represented by general formula (I)

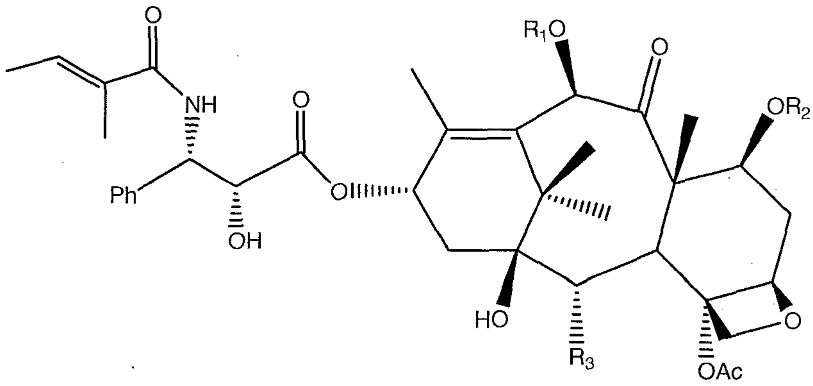

(I)

wherein,

$R_1$ is selected from the group consisting of hydrogen, TMS, TES, TBS and $-COX_1$, and $X_1$ is selected from $C_{1-5}$ alkyl;

$R_2$ is selected from the group consisting of hydrogen, substituted or unsubstituted straight or branched $C_1$-$_{15}$ alkyl, $C_2$-$_{15}$ alkenyl, $C_2$-$_{15}$ alkynyl, un-, mono- or multi-substituted aryl and heteroaryl, $-COX_2$; $-COX_3-COOX_4$; $-COX_3-CONX_4X_5$;

$R_3$ is selected from the group consisting of hydrogen, substituted or unsubstituted straight or branched $C_1$-$_{15}$ alkyl, $C_2$-$_{15}$ alkenyl, $C_2$-$_{15}$ alkynyl, un-, mono- or multi-substituted aryl and heteroaryl, $-OX_6$; $-SX_6$; $-NHX_6$; $-OCOX_6$;

and,

$X_2$, $X_3$, $X_4$, $X_5$ and $X_6$ are independently selected from the group consisting of hydrogen; substituted or unsubstituted straight or branched $C_1$-$_{15}$ alkyl, $C_2$-$_{15}$ alkenyl, $C_2$-$_{15}$ alkynyl, un-, mono- or multi-substituted aryl and heteroaryl;

the substituents for said alkyl are selected from the group consisting of hydroxy, amino, carboxyl, carbonyl, $C_1$-$_5$ alkoxy, halo, $C_{1-5}$ alkoxycarbonyl, N-$C_1$-$_5$ alkylcarbamoyl, cyano, nitro;

the substituents for said aryl and heteroaryl are selected from the group consisting of hydroxy, hydroxymethyl, halo, $C_1$-$_5$ alkyl, $C_1$-$_5$ alkoxy, $C_1$-$_5$ alkenyl, acyl, acyloxy, nitro, amino, amido, cyano, azido;

Preferred $C_1$-$_{15}$ alkenyl is selected from the group consisting of vinyl, propenyl, isopropenyl, butenyl, isobutenyl, hexenyl;

Preferred $C_1$-$_{15}$ alkynyl is selected from the group consisting of ethynyl, propinyl, isopropinyl, butynyl, isobutynyl, hexynyl;

Preferred aryl is selected from the group consisting of phenyl, naphthyl, biphenyl;

Preferred heteroaryl is selected from the group consisting of furyl, thienyl, pyridyl, benzofuryl, bipyridyl; the halo is selected from F, Cl, Br, I.

More preferred $R_1$ and $R_2$ are independently selected from the group consisting of hydrogen, formyl, acetyl, propionyl.

The symbols and terms used herein have the following meanings:

"Bz" represents benzoyl; "Ph" represents phenyl; "Ac" represents acetyl; "Et" represents ethyl ; "R" generally represents alkyl, unless otherwise indicated; "TMS" represents trimethylsilyl; "TES" represents triethylsilyl; "TBS" represents tert-butyldimethylsilyl; "DMAP" represents *p*-dimethylaminopyridine; "DMF" represents N,N-dimethylformamide; "THF" represents tetrahydrofuran; "DCC" represents dicyclohexylcarbodiimide; "PP" represents 4-pyrrolylpyridine; "r.t." represents room temperature; "TritonB" represents benzyltrimethylammonium hydroxide.

The present invention also relates to a process for the preparation of compounds according to the present invention, which is illustrated in the following schemes IIa and IIb:

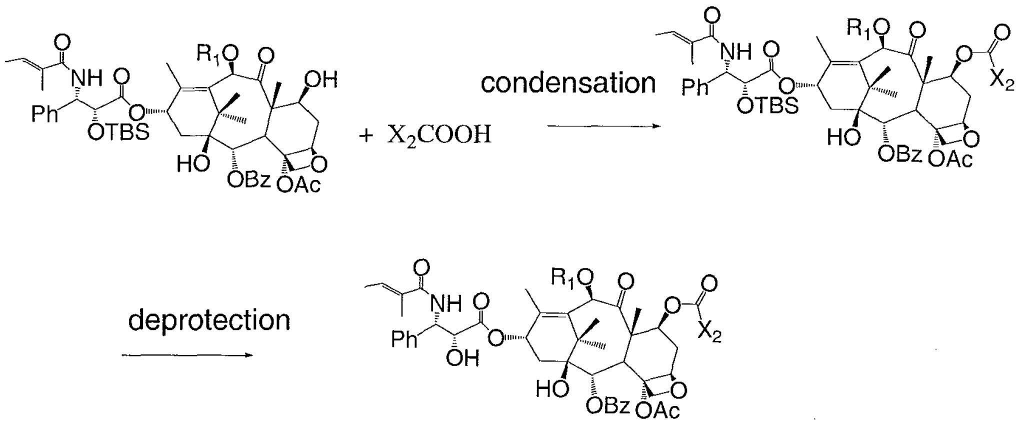


IIa

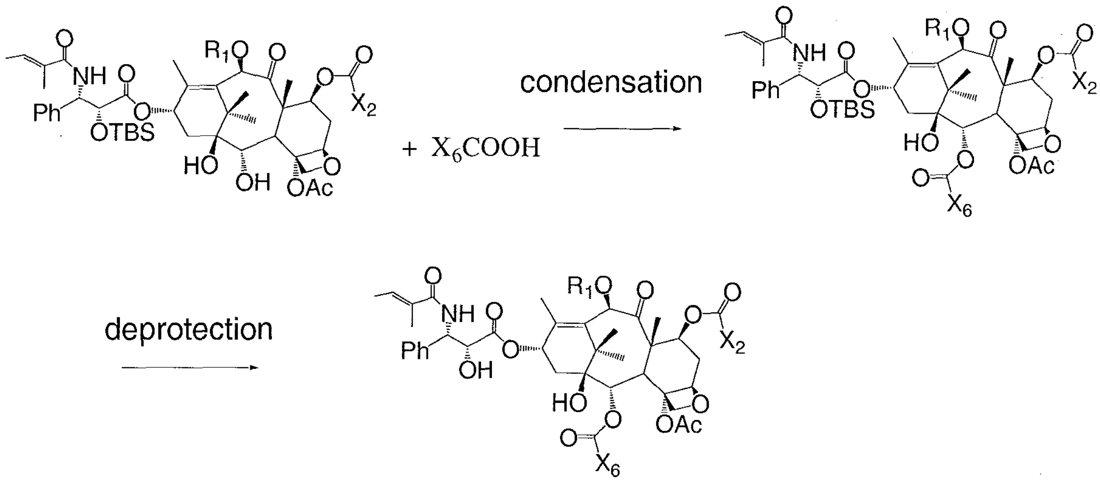


IIb

Specifically, the hydroxy at C-7 position of the cephalomannine modified or unmodified at C-10 position as starting material was condensed with a corresponding acid or acyl chloride to produce the compound of formula IIa; alternatively, after the removal of benzoyl at 2-position of the cephalomannine modified at both C-10 and C-7 positions as starting material, a key intermediate was provided, said intermediate was then condensed with a corresponding acid or acyl chloride to produce the compound of formula IIb.

The acylation reaction as mentioned above was preferably carried out in the presence of a condensation agent. Preferred condensation agents include 1,3-dicyclohexylcarbodiimide (DCC), dipyridyl carbonate(2-DPC), 1,3-diisopropyl carbodiimide (DIPC), 1-(3-dimethylaminopropyl)-3-ethylcarbodiimide (EDCI); more preferred agents are DCC, DIPC, EDCI.

The acylation reaction as mentioned above was preferably carried out in the presence of a catalyst. Preferred catalysts include tertiary amines, pyridine, 4-dimethylaminopyridine and 4-pyrrolylpyridine; more preferred catalysts are 4-dimethylaminopyridine and 4-pyrrolylpyridine.

The acylation reaction as mentioned above was preferably carried out in the presence of an organic solvent. Preferred organic solvents include dimethylsulfoxide (DMSO), toluene, methylene chloride, ethylene glycol dimethyl ether, 1,2-dichloroethane, tetrahydrofuran and N,N-dimethylformamide(DMF); more preferred organic solvents include toluene, tetrahydrofuran and N,N-dimethylformamide(DMF).

The reaction temperature ranges from 10 to 120 °C, preferably from 30 to 90 °C.

The present invention also relates to a pharmaceutical composition comprising a compound of the present invention as active ingredient. The pharmaceutical composition can be prepared according to methods well known in the art. The compound of the present invention can be formulated into any dosage forms suitable for administration to human or animals in combination with one or more pharmaceutically acceptable solid or liquid excipients and/or adjuvants. In general, the content of the compound of the present invention in the pharmaceutical composition is from 0.1 to 95 % by weight.

The compound according to the present invention or a pharmaceutical composition comprising the compound can be administered in unit dose form, the routes of administration may be intestinal or parenteral, such as oral, intravenous, intramuscularly, subcutaneous, nasal, mouth mucosa, ophthalmic, pulmonary and respiratory tract, dermal, vaginal, rectal administration etc.

The dosage forms for administration may be liquid, solid or semisolid dosage forms. The liquid dosage forms may be solutions (including true solutions and colloid solutions), emulsions (including o/w type, w/o type and multiple emulsions), suspensions, injections (including aqueous injections, powder injections and infusions), eye drops, nasal drops, lotions and liniments etc.; the solid dosage forms may be tablets (including conventional tablets, enteric tablets, buccal tablets, dispersible tablets, chewable tablets, effervescent tablets, orally disintegrating tablets), capsules (including hard capsules, soft capsules, enteric capsules), granules, granules, pellets, dropping pills, suppositories, membranes, patches, aerosols/powder inhalations, sprays, etc.; the semisolid dosage forms may be ointments, gels, pastes, etc..

The compounds of present invention can be formulated into common formulations, as well as sustained release formulations, controlled release formulations, targeting formulations and various particulate delivery systems.

To formulate the compounds of present invention into tablets, a variety of excipients well known in the art can be widely used, including diluents, binders, wetting agents, disintegrants, lubricants, glidants. The diluents may be starches, dextrins, sucrose, glucose, lactose, mannitol, sorbitol, xylitol, microcrystalline cellulose, calcium sulfate, calcium hydrogen phosphate, calcium carbonate and the like; the wetting agents may be water, ethanol, isopropanol and the like; the binders may be starch slurry, dextrins, syrup, honey, glucose solution, microcrystalline cellulose, acacia mucilage, gelatin slurry, sodium carboxymethylcellulose, methylcellulose, hydroxypropylmethylcellulose, ethyl cellulose, acrylic resins, carbomer, , polyvinylpyrrolidones, polyethylene glycols and the like; the disintegrants may be drying starches, microcrystalline cellulose, low substituted hydroxypropylcellulose, cross-linked polyvinylpyrrolidones, croscarmellose sodium, sodium carboxymethyl starch, sodium bicarbonate and citric acid, polyoxyethylene sorbitol fatty acid esters, sodium lauryl sulfate and the like; the lubricants and glidants may be talc, silicon dioxide, stearate, tartaric acid, liquid paraffin, polyethylene glycols and the like.

The tablets may be further processed to form coated tablets, e.g. sugar-coated tablets, film-coated tablets, enteric-coated tablets, or double-layer tablets and multi-layer tablets.

In order to formulate the dosing unit into capsules, the compounds according to the present invention as active ingredient can be mixed with diluents and glidants, and the mixtures are then loaded directly in hard or soft capsules. The compounds according to the present invention as active ingredient can also firstly be formulated, together with diluents, binders and disintegrants, into granules or pellets, then they are loaded in hard or soft capsules. The diluents, binders, wetting agents, disintegrants, glidants for preparing the tablets of the compounds according to the present invention can also be used for preparing the capsules of the compounds according to the present invention.

To formulate the compounds according to the present invention into injections, water, ethanol, isopropanol, propylene glycol or a mixture thereof can be used as solvent, and to which suitable amount of solubilizers, cosolvents, pH modifiers, osmotic pressure controlling agents can be added. The solubilizers or auxiliary solvents may be poloxamer, lecithin, hydroxypropyl-β-cyclodextrin, and the like; the pH modifiers may be phosphates, acetates, hydrochloric acid, sodium hydroxide, and the like; the osmotic pressure controlling agents may be sodium chloride, mannitol, glucose, phosphate, acetate, and the like. For the preparation of lyophilized powder injections, mannitol, glucose, etc. can also be added as support agents.

In addition, the colorants, preservatives, fragrant agents, flavoring agents or other additives may also be added to the pharmaceutical formulations if necessary.

According to the present invention, the compounds of general formula (I) show significant biological activity in pharmacological screening assay, and are useful antineoplastic agents. They can be used to inhibit the growth of tumors in the body of animals including humans.

According to the *in vitro* pharmacological experimental, it has been found that the compounds according to the present invention exhibit potent inhibitory effect on sensitive tumors, wherein the compound with the strongest activity exhibits comparable activity to taxol for the sensitive tumors; however, its activity is dozens of times as high as that of taxol for the drug resistant tumors arising from the overexpression of P-glycoproteins. The compounds according to the present invention are also effective for the drug resistant tumors rising from mutations of the amino acids residues of tubulins, and their activities are stronger than taxol.

The *in vivo* experiment revealed that the growth inhibition effect of the compounds according to the present invention on human pulmonary adenocarcinoma A549 nude mice xenograft tumor is stronger than that of taxol.

In order to achieve the purpose of treatment and enhance the effect of treatment, the medicament of the present invention or pharmaceutical composition can be administrated by any known manner for administration.

The dose of the compounds or pharmaceutical compositions of the present invention will vary in a wide range depending on the nature and severity of the diseases to be prevented or treated, individual condition of the patients or animals, administration routes, dosage forms, and the like. In general, the suitable daily dose range of the compounds according to the present invention is from 0.001 to 150 mg/Kg body weight, preferably from 0.1 to 100 mg/Kg body

weight, more preferably from 1 to 60 mg/Kg body weight, most preferably from 2 to 30 mg/Kg body weight. Said dose may be administrated as a single dose unit or divided dose units, depending on the clinical experience of the physicians and the dose regimen including the use of other therapeutic manners.

The compounds or compositions of the present invention may be administrated alone or in combination with other therapeutic agents or the agents for symptomatic treatment. When a compound of the present invention is synergistic with other therapeutic agents, its dose should be adjusted according to the practical condition.

## Examples

The following examples serve to illustrate the present invention without limiting the scope thereof.

**Example 1: Preparation of 10-deacetyl-cephalomannine**

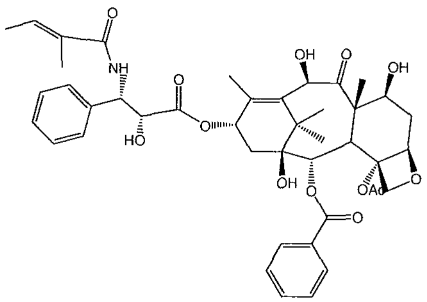

Step 1: 2'-tert-butyldimethylsilyl-cephalomannine

Cephalomannine (600 mg, 0.721mmol) was dissolved in 5 ml DMF, then imidazole (245.5 mg, 3.61 mmol) and tert-butyldimethylsilyl chloride (543.5 mg, 3.61 mmol) were added. After reacting at 70 °C for 5 h, 10 ml of saturated aqueous $NaHCO_3$ was added. The mixture was extracted with ethyl acetate (3 x 50 ml). The ethyl acetate layers were combined, and dried over anhydrous sodium sulfate. The ethyl acetate layer was evaporated to dryness. A silica gel column chromatography (petroleum ether : ethyl acetate = 5 : 1) was carried out to yield the title product (637 mg, 93.4%).

Step 2: 2'-tert-butyldimethylsilyl-10-deacetyl-cephalomannine

2'-tert-butyldimethylsilyl-cephalomamine (98 mg, 0.103mmol) was dissolved in 6 ml ethanol, then 85% hydrazine hydrate (0.625 ml) was added. After reacting at room temperature for 2 h, 10 ml of saturated aqueous $NH_4Cl$ was added. The mixture was exacted with ethyl acetate (3 x 50 ml). The ethyl acetate layers were combined, and dried over anhydrous sodium sulfate. The ethyl acetate layer was evaporated to dryness. Then a silica gel column chromatography (petroleum ether : ethyl acetate = 2: 1) was carried out to yield the title product (79.4 mg, 84.8%).

Step 3: 1**0**-deacetyl-cephalomannine

2'-tert-butyldimethylsilyl-10-deacetyl-cephalomannine (32 mg, 0.035mmol) was dissolved in 1.4 ml of acetonitrile, pyridine (0.683 ml) and HF (0.375 ml) was added sequentially. After reacting at room temperature for 24 h, 30 ml of ethyl acetate was added. The mixture was washed with saturated aqueous $NaHCO_3$ (2 x 20 ml), and the aqueous layer was extracted with ethyl acetate (3 x 50 ml). The ethyl acetate layers were combined, and dried over anhydrous sodium sulfate. The ethyl acetate layer was then evaporated to dryness. And a silica gel column chromatography (chloroform : methanol = 20: 1) was carried out to yield the title product (25.7 mg, 91.92%).
$^1H$ 300M ($CDCl_3$): δ 8.10 (2H, d, *J* = 7.2 Hz), 7.61 (1H, t, *J* =7.2 Hz), 7.50 (2H, t, *J* = 7.2 Hz), 7.28-7.39 (5H, m), 6.65 (1H, d, *J* = 9.0 Hz), 6.44 (1H, dq, *J* = 7.2,1.4 Hz), 6.16 (1H, t, *J* = 8.1 Hz), 5.66 (1H, d, *J* = 6.9 Hz), 5.59 (1H, dd, *J* = 8.9, 2.7 Hz), 5.18 (1H, s), 4.91 (1H, d, *J* = 7.8 Hz), 4.91 (1H, brs), 4.39 (1H, d, *J* = 8.7 Hz), 4.24 (1H, brs), 4.19 (1H, d, *J* = 8.4 Hz), 3.87 (1H, d, *J* = 6.9 Hz), 3.72 (1H, brs), 2.53-2.62 (1H, m), 2.34 (3H, s), 2.24 (2H, d, *J* = 9.0 Hz), 1.82-1.88 (1H, m), 1.82 (3H, s), 1.76 (3H, s), 1.72 (3H, d, *J* = 7.5 Hz), 1.21 (3H, s), 1.11 (3H, s). ESI-MS: *m*/*z* $[M+Na]^+$ 812.3.

Examples 2-4 were prepared according to the following scheme

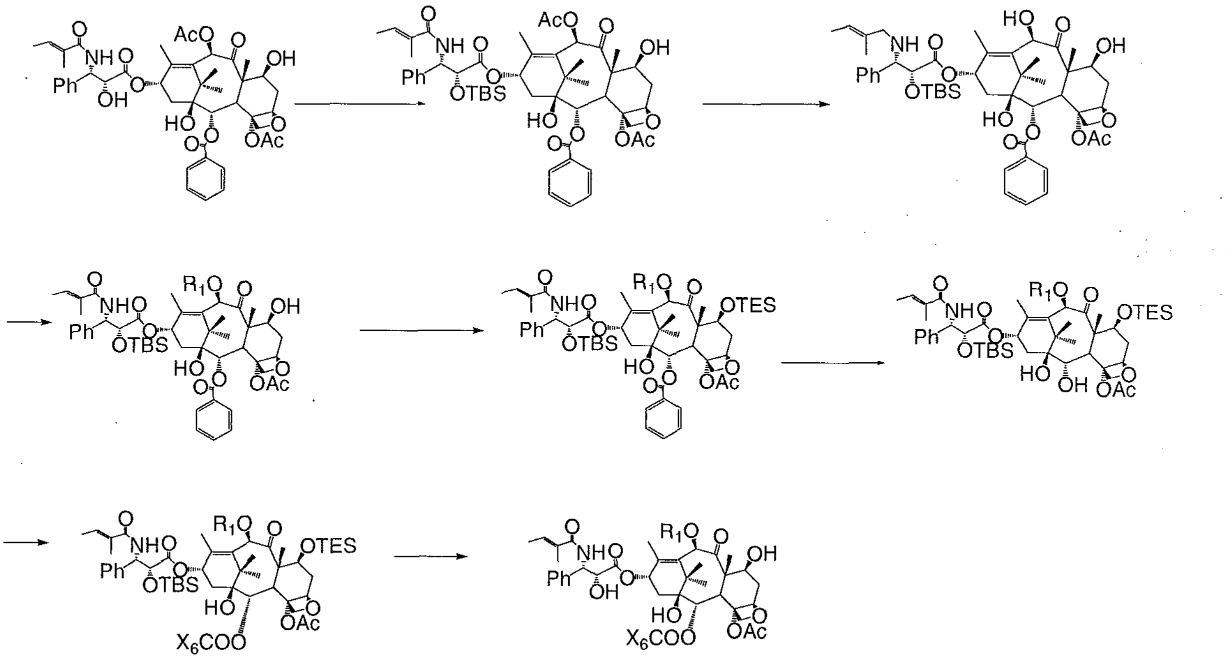

**Example 2: Preparation of 2-(3-azidobenzoyl)-10-propionyl-cephalomannine**

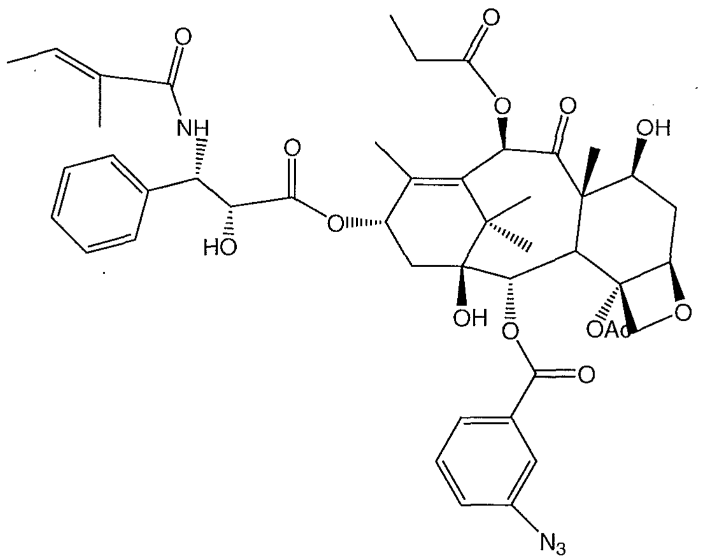

Steps 1-2: Being the same as procedures described in steps 1-2 of Example 1.

Step 3: 2'-tert-butyldimethylsilyl-10-propionyl-cephalomannine

2'-tert-butyldimethylsilyl-10-deacetyl-cephalomannine (155 mg, 0.171mmol) was dissolved in 5 ml THF, then

$CeCl_3$(8.4 mg) was added, cooling in ice bath. Propionic anhydride (0.22 ml, 1.71mmol) was added. After reacting at 30 °C for 2 h, 300 ml ethyl acetate was added, and washed with saturated aqueous $NaHCO_3$ (2 x 50 ml) and 50 ml saturated NaCl solution. The aqueous layer was extracted with ethyl acetate (150 ml). The ethyl acetate layers were combined, dried over anhydrous sodium sulfate, and filtered. The ethyl acetate layer was evaporated to dryness. And a silica gel column chromatography (acetone : petroleum ether = 1: 2) was carried out to yield the title product (148.2 mg, 97%).

Step 4: 2'-tert-butyldimethylsilyl-7-triethylsilyl-10-propionyl-cephalomannine

2'-tert-butyldimethylsilyl-10-propionyl-cephalomannine (193 mg, 0.201mmol) was dissolved in 2 ml of DMF, then imidazole (73 mg, 1.07mmol) and triethylsilyl chloride (0.135 ml) were added. After reacting at room temperature for 10 h, saturated aqueous $NaHCO_3$ (10 ml) was added. The mixture was extracted with ethyl acetate (3 x 50 ml). The ethyl acetate layers were combined, and dried over anhydrous sodium sulfate. The ethyl acetate layer was evaporated to dryness. And a silica gel column chromatography (petroleum ether : ethyl acetate = 5: 1) was carried out to yield the title product (198 mg, 91.7%).

Step 5: 2'-tert-butyldimethylsilyl-2-debenzoyl-7-triethylsilyl-10-propionyl-cephalomannine

2-tert-butyldimethylsilyl-7-triethylsilyl-10-propionyl-cephalomannine (168 mg, 0.156mmol) was dissolved in 7 ml of methylene chloride. After cooling at -28 °C in ice-methanol bath, TritonB (0.138 ml, 0.313mmol) was added dropwise. After reacting for 15 minutes, 20 ml of methylene chloride was added. The mixture was washed with saturated aqueous ammonium chloride (20 ml). The aqueous layer was extracted with ethyl acetate (2 x 50 ml). The organic layer was washed with 20 ml of saturated NaCl solution, then dried over anhydrous sodium sulfate, and filtered. The organic layer was evaporated to dryness. A silica gel column chromatography (n-hexane : ethyl acetate : acetone = 8: 3: 1) was carried out to yield the title product (110 mg, 73%).

Step 6: 2-(3-azidobenzoyl)-10-propionyl-cephalomannine

2'-tert-butyldimethylsilyl-2-debenzoyl-7-triethylsilyl-10-propionyl-cephalomannine (35.7 mg, 0.0368 mmol) was dissolved in 1 ml of toluene, then *m*-azidobenzoic acid (62.5 mg, 0.383mmol), PP (5.7 mg, 0.0383mmol) and DCC (79 mg, 0.383mmol) were added. After reacting at 65 °C for 10 h, 0.1 ml of methanol was added, followed by filtration. The solid was washed with ethyl acetate. The filtrates were combined and evaporated to dryness. Then a silica gel column chromatography (petroleum ether : ethyl acetate = 8: 1) was carried out. The resulting product was contaminated with impurities such as DCU, and said product was deprotected, without a further purification, as follows. Said product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml). The aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane : ethyl acetate : acetone = 8: 3: 2) was carried out to yield the title product (20.3 mg, 62.3% of total yield over two steps).

$^1H$ 300M ($CDCl_3$): δ 7.89 (1H, d, *J* = 8.1 Hz), 7.80 (1H, m), 7.48 (1H, t, *J* = 7.8 Hz), 7.31-7.42 (5H, m), 7.24 (1H, ddd, *J* = 1.2, 2.4, 8.1 Hz), 6.49 (1H, d, *J* = 8.7 Hz), 6.40 (1H, dq, *J* = 1.5, 6.9 Hz), 6.28 (1H, s), 6.17 (1H, t, *J* = 8.1 Hz), 5.66 (1H, d, *J* = 6.9 Hz), 5.58 (1H, dd, *J* = 2.7, 8.7 Hz), 4.94 (1H, d, *J* = 7.8 Hz), 4.68 (1H, brs), 4.40 (1H, m), 4.30 (1H, d, *J* = 8.4 Hz), 4.17 (1H, d, *J* = 8.4 Hz), 3.80 (1H, d, *J* = 6.9 Hz), 3.63 (1H, brs), 2.47-2.62 (3H, m), 2.37 (3H, s), 2.31 (2H, overlap), 1.82-1.95 (1H, m), 1.78-1.80 (6H, brs), 1.72 (3H, dd, *J* = 1.5, 6.9 Hz), 1.67 (3H, s), 1.25 (3H, s), 1.23 (3H, t, *J* = 7.8 Hz), 1.14 (3H, s). ESI-MS: m/z $[M+Na]^+$ 909.4.

**Example 3: Preparation of 2-(3-methoxybenzoyl)-10-propionyl-cephalomannine**

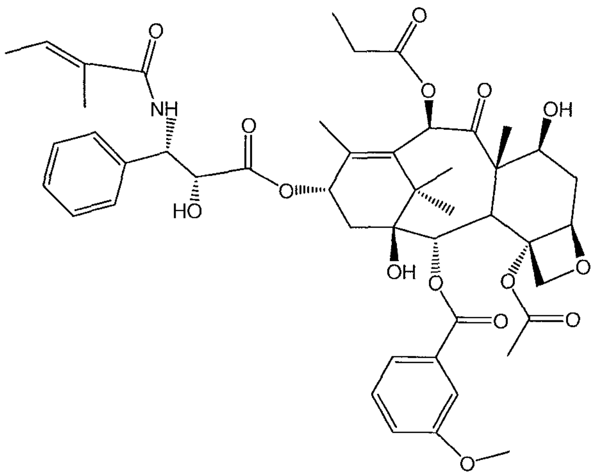

Steps 1-5: Being the same as procedures described in steps 1-5 of Example 2.

Step 6: 2-(3-methoxybenzoyl)-10-propionyl-cephalomannine

2'-tert-butyldimethylsilyl-2-debenzoyl-7-triethylsilyl-10-propionyl-cephalomanni ne (37.2 mg, 0.0383mmol) was dissolved in 1 ml of toluene, then *m*-methoxybenzoic acid (58.3 mg, 0.383mmol), PP (5.7 mg, 0.0383mmol) and DCC (79 mg, 0.383mmol) were added. After reacting at 65 °C for 24 h, 0.1 ml of methanol was added, followed by filtration. The solid was washed with ethyl acetate. The filtrates were combined and evaporated to dryness. Then a silica gel column chromatography (petroleum ether : ethyl acetate = 8: 1) was carried out. The resulting product was contaminated with impurities such as DCU, and said product was deprotected, without a further purification, as follows. The product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml). The aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane : ethyl acetate : acetone = 8: 3: 2) was carried out to yield the title product (21.0 mg, 62.5% of total yield over two steps).
$^1$H 300M ($CDCl_3$): δ 7.71 (1H, d, *J* = 8.1 Hz), 7.62-7.69 (1H, m), 7.29-7.43 (6H, m), 7.15(1H, ddd, *J* = 0.9, 2.7, 8.3 Hz), 6.51 (1H, d, *J* = 8.7 Hz), 6.42 (1H, dq, *J* = 1.5, 7.2 Hz), 6.39 (1H, s), 6.19 (1H, t, *J* = 7.5 Hz), 5.66 (1H, d, *J* = 6.9 Hz), 5.59 (1H dd, *J* = 2.7, 8.6 Hz), 4.94 (1H, d, *J* = 7.5 Hz), 4.69 (1H, brs), 4.36-4.44 (1H, m), 4.33 (1H, d, *J* =8.4 Hz), 4.17 (1H, d, *J* = 8.4 Hz), 3.88 (3H, s), 3.79 (1H, d, *J* = 6.9 Hz), 3.63 (1H, brs), 2.45-2.63 (3H, m), 2.33 (3H, s), 2.26 (2H, dd, *J* = 3.0, 9.0 Hz), 1.86-1.92 (1H, m), 1.80 (3H, pseud-t, *J* =1.2 Hz), 1.79 (3H, d, *J* =1.2 Hz), 1.72 (3H, dd, *J* = 1.2, 6.9Hz), 1.67 (3H, s), 1.25 (3H, s), 1.23 (3H, t, *J* = 7.5 Hz), 1.14 (3H, s). ESI-MS: *m*/*z* $[M+Na]^+$ 898.4.

**Example 4: Preparation of 10-propionyl-cephalomannine**

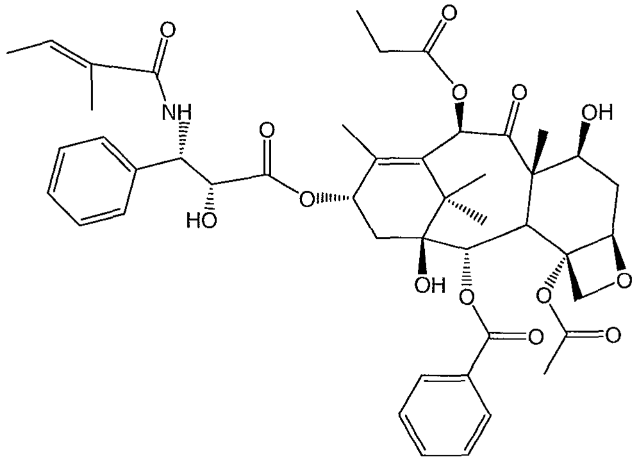

Steps 1-3: Being the same as procedures described in steps 1-3 of Example 2.

Step 4: 10-propionyl-cephalomannine

2'-tert-butyldimethylsilyl-10-propionyl-cephalomannine (26 mg, 0.027mmol) was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml). The aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (chloroform: methanol = 20: 1) was carried out to yield the title product (21.1 mg, 92%).
$^1$H 300M ($CDCl_3$): δ 8.11 (1H, d, *J* = 6.9 Hz), 7.61 (1H, t, *J* = 7.5 Hz), 7.50 (2H, t, *J* = 7.5 Hz), 7.31-7.42 (5H, m), 6.54 (1H, d, *J* = 9.0 Hz), 6.43 (1H, dq, *J* = 1.2, 6.9 Hz), 6.28 (1H, s), 6.20 (1H, t, *J* = 7.5 Hz), 5.66 (1H, d, *J* = 7.2 Hz), 5.61 (1H, dd, *J* = 3.0, 8.7 Hz), 4.93 (1H, dd, *J* = 2.1, 9.5 Hz), 4.70 (1H, d, *J* = 2.4 Hz), 4.36-4.42 (1H, m), 4.29 (1H, d, *J* = 8.7 Hz), 4.18 (1H, d, *J* = 8.4), 3.79 (1H, d, *J* = 7.2), 3.66 (1H, brs), 2.47-2.62 (3H, m), 2.35 (3H, s), 2.25-2.34 (2H, m), 1.86-1.91 (1H, m), 1.80 (3H, s), 1.79 (3H, s), 1.72 (3H, dd, *J* = 1.2, 6.9 Hz), 1.67 (3H, s), 1.25 (3H, s), 1.23 (3H, t, *J* = 7.5 Hz), 1.14 (3H, s). ESI-MS: *m/z* $[M+Na]^+$ 884.4.

**Example 5: Preparation of 2-(3-azidobenzoyl)-cephalomannine**

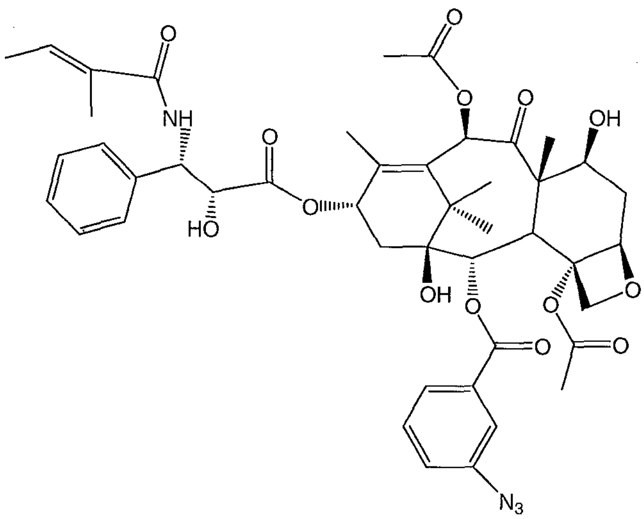

Step 1: Being the same as the procedure described in Step1 of Example 1.

Step 2: 2-tert-butyldimethylsilyl-2-debenzoyl-cephalomannine

2'-tert-butyldimethylsilyl-cephalomannine (351 mg, 0.329mmol) was dissolved in 7 ml of methylene chloride, cooling at -28 °C in ice-methanol bath, then TritonB (0.308 ml) was added dropwise. After reacting for 15 minutes, 20 ml of methylene chloride was added. The organic layer was washed with 20 ml of saturated ammonium chloride solution. The aqueous layer was extracted with ethyl acetate (2 x 50 ml). The organic layer was washed with 20 ml of saturated NaCl solution. The organic layer was dried over anhydrous sodium sulfate, and filtered. The organic layer was then evaporated to dryness. A silica gel column chromatography (n-hexane : ethyl acetate : acetone = 8: 3: 1) was carried out to yield the title product (209 mg, 67.4%).

Step 3: 2-(3-azidobenzoyl)-cephalomannine

2'-tert-butyldimethylsilyl-2-debenzoyl-cephalomannine (62 mg, 0.0662 mmol) was dissolved in 1 ml of toluene, then *m*-azidobenzoic acid (121.4 mg, 0.744 mmol), PP (11 mg, 0.0742 mmol) and DCC (153.51 mg, 0.744 mmol) were added. After reacting at 65 °C for 10 h, 0.1 ml methanol was added, then filtered. The solid was washed with ethyl acetate. The filtrate was combined and evaporated to dryness. Then a silica gel column chromatography (petroleum ether : ethyl acetate = 8: 1) was carried out. The resulting product was contaminated with impurities such as DCU, and said product was deprotected, without a further purification, as follows. Said product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml), the aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane : ethyl acetate : acetone = 8: 3: 2) was carried out to yield the title product (43 mg, 83.4% of total yield over two steps).
$^1H$ 300M ($CDCl_3$): δ 7.89 (1H, d, *J* = 8.1 Hz), 7.79 (1H, m), 7.48 (1H, t, *J* = 7.8 Hz), 7.31-7.40 (5H, m), 7.22-7.25 (1H, m), 6.49 (1H, d, *J* = 8.7 Hz), 6.41 (1H, dq, *J* = 1.5, 6.9 Hz), 6.27 (1H, s), 6.17 (1H, t, J = 8.5 Hz), 5.66 (1H, d, *J* = 7.2 Hz), 5.58 (1H, dd, *J* =2.7, 8.7 Hz), 4.94 (1H, d, *J* = 7.5 Hz), 4.68 (1H, d, *J* = 2.7 Hz), 4.39 (1H, dd, *J* = 6.9, 9.45 Hz), 4.30 (1H, d, *J* = 8.4 Hz), 4.16 (1H, d, *J* = 8.4 Hz), 3.79 (1H, d, *J* = 7.2 Hz), 2.48-2.62 (1H, m), 2.34 (3H, s), 2.30 (2H, d, *J* = 9.0 Hz), 2.24 (3H, s), 1.82-1.91 (1H, m), 1.79 (6H, s), 1.73 (3H, d, *J* = 6.9 Hz), 1.67 (3H, s), 1.25 (3H, s), 1.14 (3H, s). ESI-MS: *m*/*z* $[M+Na]^+$ 895.4.

**Example 6: Preparation of 2-(3-methoxybenzoyl)-cephalomannine**

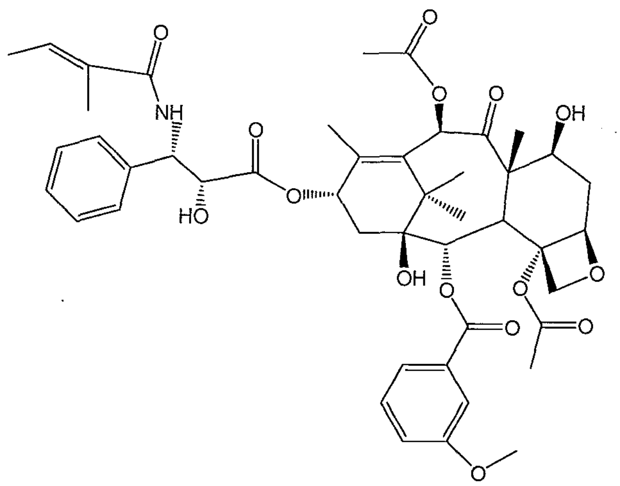

Step 1: Being the same as the procedure described in Step 1 of Example 1.

Step 2: Being the same as the procedure described in Step2 of Example 5.

Step 3: 2-(3-methoxybenzoyl)-cephalomannine

2'-tert-butyldimethylsilyl-2-debenzoyl-cephalomannine (56 mg, 0.0598mmol) was dissolved in 1 ml of toluene, *m*-methoxybenzoic acid (113 mg, 0.742 mmol), PP (11 mg, 0.0742 mmol) and DCC (153.51 mg, 0.744 mmol) were added. After reacting at 65 °C for 10 h, 0.1 ml methanol was added, then filtered. The solid was washed with ethyl acetate. The filtrates were combined and evaporated to dryness. Then a silica gel column chromatography (petroleum ether : ethyl acetate = 8: 1) was carried out. The resulting product was contaminated with impurities such as DCU, and said product was deprotected, without a further purification, as follows. Said product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml), the aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane : ethyl acetate : acetone = 8: 3: 2) was carried out to yield the title product (37.3 mg, 64.5% of total yield over two steps).
$^1$H 300M ($CDCl_3$): δ 7.70 (1H, d, *J* = 7.5 Hz), 7.68 (1H, m), 7.30-7.42 (6H, m), 7.13 (1H, dd, *J* = 2.7, 7.5 Hz), 6.55 (1H, d, *J* = 8.7 Hz), 6.42 (1H, dq, *J* = 1.2, 6.9 Hz), 6.26 (1H, s), 6.18 (1H, t, *J* = 7.8 Hz), 5.65 (1H, d, *J* = 6.9 Hz), 5.58 (1H, dd, *J* = 3.0, 8.55 Hz), 4.93 (1H, d, *J* = 7.5 Hz), 4.69 (1H, d, *J* = 3.0 Hz), 4.38 (1H, dd, *J* = 6.6, 11.0 Hz), 4.31 (1H, d, *J* = 8.4 Hz), 4.17 (1H, d, *J* = 8.4 Hz), 3.86 (3H, s), 3.77 (1H, d, *J* = 6.9 Hz), 2.48-2.62 (1H, m), 2.32 (3H, s), 2.28 (2H, d, *J* = 7.2 Hz), 1.86-1.90 (1H, m), 1.79 (3H, s), 1.78 (3H, s), 1.71 (3H, d, *J* = 6.9 Hz), 1.66 (3H, s), 1.25 (3H, s), 1.14 (3H, s). ESI-MS: *m*/*z* $[M+Na]^+$ 884.4.

Examples 7-14 were prepared according to the following scheme:

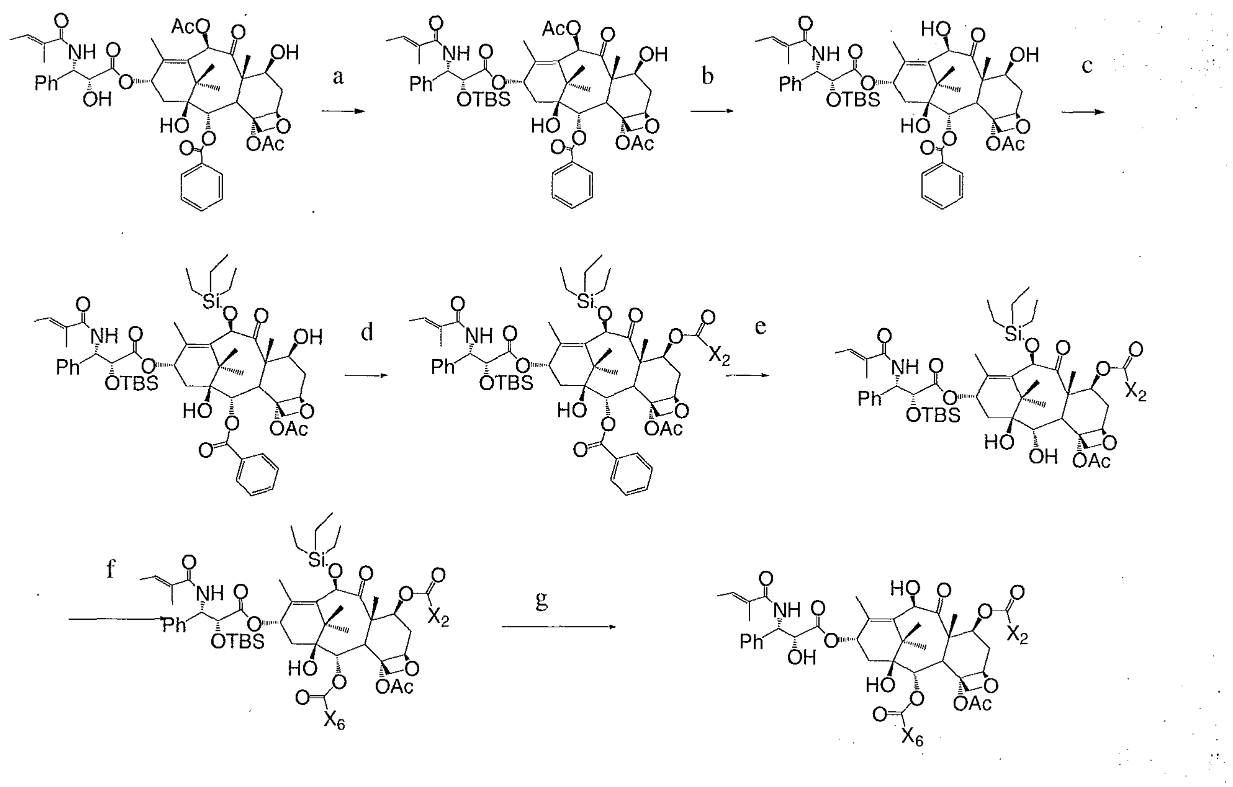


**Example 7: Preparation of 2-(3-methoxybenzoyl)-7-propionyl-10-deacetyl-cephalomannine**

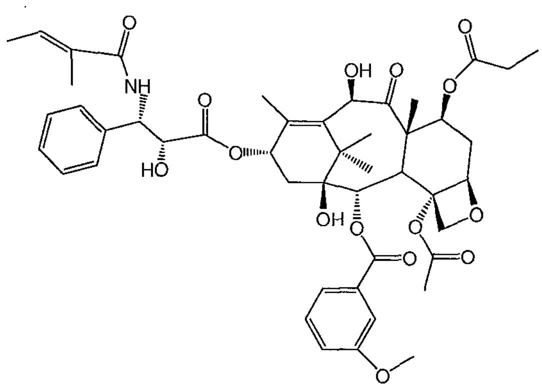

Steps 1-2: Being the same as the procedures described in Steps 1-2 of Example 1.

Step 3: 2'-tert-butyldimethylsilyl-10-triethylsilyl-cephalomannine

2'-tert-butyldimethylsilyl-10-deacetyl-cephalomannine (300 mg, 0.332 mmol) was dissolved in 10 ml of THF under $N_2$, then cooled in ice-salt bath at -10°C, and N,O-bis(triethylsilyl)-trifluoroacetamide (1 ml) and LHMDS (3ul) were added. After reacting for 10 minutes, saturated aqueous $NaHCO_3$ (10 ml) was added, then extracted with ethyl acetate (3 x 50 ml). The ethyl acetate layers were combined, and dried over anhydrous sodium sulfate. The ethyl acetate layer was evaporated to dryness. And a silica gel column chromatography (petroleum ether : ethyl acetate = 5: 1) was carried out to yield the title product (314 mg, 92.6%).

Step 4: 2'-tert-butyldimethylsilyl-10-triethylsilyl-7-propionyl-cephalomannine

2'-tert-butyldimethylsilyl-10-triethylsilyl-cephalomannine (300 mg, 0.171 mmol) was dissolved in 5 ml of THF, then cooled in ice bath, and propionic anhydride (0.40 ml) was added. After reacting at room temperature for 20 h, 300 ml of ethyl acetate was added, then washed with saturated aqueous $NaHCO_3$ (2 x 50 ml) and saturated NaCl solution (50 ml). The aqueous layer was extracted with ethyl acetate (150 ml). The ethyl acetate layer was combined, dried over anhydrous sodium sulfate, and filtered. The ethyl acetate layer was evaporated to dryness. And a silica gel column chromatography (acetone : petroleum ether = 1: 2) was carried out to yield the title product (301.0 mg, 95.1%).

Step 5: 2'-tert-butyldimethylsilyl-2-debenzoyl-10-triethylsilyl-7-propionyl-cephalomannine

2'-tert-butyldimethylsilyl-10-triethylsilyl-7-propionyl-cephalomannine (168 mg, 0.156 mmol) was dissolved in 7 ml of methylene chloride, then cooled in ice-methanol bath at -28 °C, and TritonB (0.138 ml , 0.313 mmol) was added dropwise. After reacting for 15 minutes, 20 ml of methylene chloride was added, then washed with 20 ml of saturated ammonium chloride solution. The aqueous layer was extracted with ethyl acetate (2 x 50 ml). The organic layer was washed with 20 ml of saturated NaCl solution. The organic layer was dried over anhydrous sodium sulfate, and filtered. The organic layer was evaporated to dryness. A silica gel column chromatography (n-hexane : ethyl acetate : acetone = 8: 3: 1) was carried out to yield the title product (110 mg, 73%).

Step 6: 2-(3-methoxybenzoyl)-7-propionyl-10-deacetyl-cephalomamine

2' -tert-butyldimethylsilyl-2-debenzoyl-10-triethylsilyl-7-propionyl-cephalomannin e (26 mg, 0.0268 mmol) was dissolved in 1 ml of toluene, *m*-methoxybenzoic acid (40.76 mg, 0.268 mmol), PP (2.38 mg, 0.016 mmol) and DCC (55.28 mg, 0.268 mmol) were added. After reacting at 65 °C for 10 h, 0.1 ml methanol was added, then filtered. The solid was washed with ethyl acetate. The filtrates were combined and evaporated to dryness.Then a silica gel column chromatography (petroleum ether : ethyl acetate = 8: 1) was carried out. The resulting product was contaminated with impurities such as DCU, and said product was deprotected, without a further purification, as follows. The product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml), the aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane : ethyl acetate : acetone = 8: 3: 2) was carried out to yield the title product (18.5 mg, 72.9% of total

yield over two steps).
$^1$H 300M ($CDCl_3$): δ 7.70 (1H, d, *J* = 7.2 Hz), 7.62 (1H, brs), 7.31-7.43 (6H, m), 7.15 (1H, dd, *J* = 2.1, 8.1 Hz), 6.61 (1H, d, *J* = 8.7 Hz), 6.44 (1H, q, *J* = 6.9 Hz), 6.15 (1H, t, *J* = 8.4 Hz), 5.66 (1H, d, *J* = 6.6 Hz), 5.59 (1H, dd, *J* = 2.4, 9.0 Hz), 5.45 (1H, dd, *J* = 7.2, 10.2 Hz), 5.28 (1H, s), 4.91 (1H, d, *J* = 8.4 Hz), 4.69 (1H, d, *J* = 2.1 Hz), 4.35 (1H, d, *J* = 8.4 Hz), 4.20 (1H, d, *J* = 8.7 Hz), 3.97 (1H, overlapped), 3.96 (1H, d, *J* = 6.9 Hz), 3.87 (3H, s), 3.6 (1H, brs), 2.46-2.56 (1H, m), 2.34 (3H, s), 2.25 (2H, m), 2.24 (2H, q, *J* =7.8 Hz), 1.91-1.95 (1H, m), 1.86 (3H, s), 1.81 (6H, brs), 1.73 (3H, d, *J* = 6.9 Hz), 1.20 (3H, s), 1.09 (3H, t, *J* = 7.5 Hz), 1.08 (3H, s). ESI-MS: *m*/*z* $[M+Na]^+$ 898.3, $[M+K]^+$ 914.3.

**Example 8: Preparation of 2-(3-azidobenzoyl)-7-propionyl-10-deacetyl -cephalomannine**

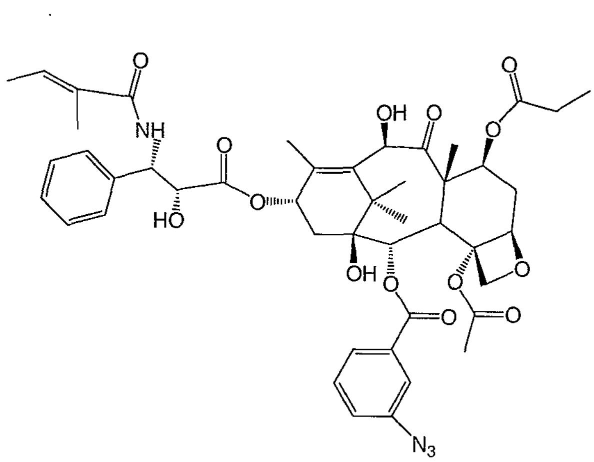

Steps 1-5: Being the same as the procedures described in Steps 1-5 of Example 7.

Step 6: 2-(3-azidobenzoyl)-7-propionyl-10-deacetyl-cephalomannine

2'-tert-butyldimethylsilyl-2-debenzoyl-10-triethylsilyl-7-propionyl-cephalomannin e (20 mg,0.0206 mmol) was dissolved in 1 ml of toluene, and *m*-azidobenzoic acid (33.6 mg, 0.206 mmol), PP (1.8 mg, 0.0124 mmol) and DCC (42.5 mg, 0.206 mmol) were added. After reacting at 65 °C for 10 h, 0.1 ml methanol was added, then filtered. The solid was washed with ethyl acetate. The filtrates were combined and evaporated to dryness, followed by a silica gel column chromatography (petroleum ether : ethyl acetate = 8: 1). The resulting product was contaminated with impurities such as DCU, and said product was deprotected, without a further purification, as follows. The product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml), the aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane: ethyl acetate : acetone = 8: 3: 2) was carried out to yield the title product (15.0 mg, 76.5% of total yield over two steps).
$^1$H ($CDCl_3$, 300MHz): δ 7.89 (1H, d, *J* = 7.2 Hz), 7.79 (1H, brs), 7.49 (1H, t, *J* = 7.2 Hz), 7.21-7.40 (6H, m), 6.57 (1H, d, *J* = 8.7 Hz), 6.42 (1H, q, *J* = 6.7 Hz), 6.14 (1H, t, *J* = 8.9 Hz), 5.66 (1H, d, *J* = 7.5 Hz), 5.59 (1H, dd, *J* = 2.7, 9.0 Hz), 5.46 (1H, dd, *J* = 7.5, 10.5 Hz), 5.29 (1H, s), 4.92 (1H, d, *J* = 8.5 Hz), 4.68 (1H, d, *J* = 2.7 Hz), 4.32 (1H, d, *J* = 8.1 Hz), 4.19 (1H, d, *J* = 8.1 Hz), 3.98 (1H, d, *J* = 7.2 Hz), 3.97 (1H, overlapped), 3.60 (1H, brs), 2.52 (1H, ddd, *J* = 7.3, 9.5, 15.0 Hz), 2.34 (3H, s), 2.21-2.34 (2H, m), 2.25 (2H, q, *J* = 7.6 Hz), 1.92 (1H, m), 1.84 (3H, s), 1.83 (3H, s), 1.80 (3H, s), 1.72 (3H, d, J = 6.9 Hz), 1.21 (3H, s), 1.09 (3H, t, *J* = 7.5 Hz), 1.08 (3H, s). ESI-MS: *m*/*z* $[M+Na]^+$ 909.3, $[M+K]^+$ 925.3.

**Example 9: 2-(3-chlorobenzoyl)-7-propionyl-10-deacetyl-cephalomannine**

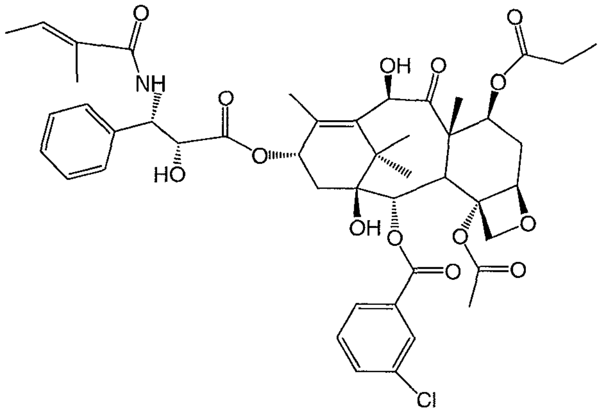

Steps 1-5: Being the same as the procedures described in Steps 1-5 of Example 7.

Step 6: 2-(3-chlorobenzoyl)-7-propionyl-10-deacetyl-cephalomannine

2' -tert-butyldimethylsilyl-2-debenzoyl-10-triethylsilyl-7-propionyl-cephalomannin e (23 mg, 0.0237 mmol) was dissolved in 1 ml of toluene, *m*-chlorobenzoic acid (37.1 mg, 0.237 mmol), PP (2.1 mg, 0.014 mmol) and DCC (48.9 mg, 0.237 mmol) were added. After reacting at 65 °C for 10 h, 0.1 ml methanol was added, then filtered. The solid was washed with ethyl acetate. The filtrates were combined and evaporated to dryness, followed by a silica gel column chromatography (petroleum ether : ethyl acetate = 8: 1). The resulting product was contaminated with impurities such as DCU, and said product was deprotected, without a further purification, as follows. The product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml), the aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane : ethyl acetate : acetone = 8: 3: 2) was carried out to yield the title product (17.5 mg, 78.3% of total yield over two steps). $^1H$ ($CDCl_3$, 500MHz): δ 8.10 (1H, s), 8.00 (1H, d, *J* = 7.5 Hz), 7.59 (1H, dd, *J* = 1.5, 8.0 Hz), 7.46 (1H, t, *J* = 8.0 Hz), 7.30-7.414 (5H, m), 6.59 (1H, d, *J* = 8.5 Hz), 6.44 (1H, q, *J* = 7.0 Hz), 6.13 (1H, t, *J* = 9.0 Hz), 5.62 (1H, d, *J* = 7.5 Hz), 5.59 (1H, dd, *J* = 2.5, 9.0 Hz), 5.44 (1H, dd, *J* = 7.5, 10.5 Hz), 5.28 (1H, s), 4.92 (1H, d, *J* = 8.5 Hz), 4.69 (1H, d, *J* = 1.5 Hz), 4.30 (1H, d, *J* = 8.5 Hz), 4.18 (1H, d, *J* = 9.0 Hz), 3.97 (1H, d, *J* = 6.5 Hz), 3.97 (1H, overlapped), 3.60 (1H, brs), 2.51 (1H, ddd, *J* = 7.3, 9.5, 15.0 Hz), 2.34 (3H, s), 2.28 (2H, m, overlapped), 2.25 (2H, q, J = 7.6 Hz) 1.92 (1H, m), 1.84 (3H, s), 1.82 (3H, s), 1.81 (3H, s), 1.73 (3H, d, *J* = 6.9 Hz), 1.20 (3H, s), 1.09 (3H, t, *J* = 7.5 Hz), 1.07 (3H, s). ESI-MS: *m*/*z* $[M+Na]^+$ 902.3, $[M+K]^+$ 918.3.

**Example 10: Preparation of 2-(3-methyl-2-butenoyl)-7-propionyl-10-deacetyl -cephalomannine**

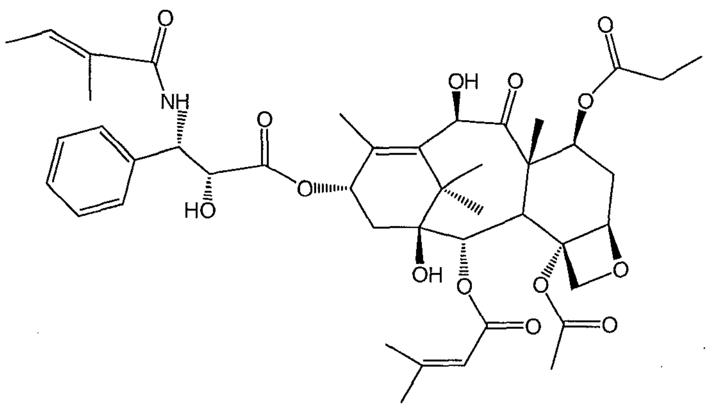

Steps 1-5: Being the same as the procedures described in Steps 1-5 of Example 7.

Step 6: 2-(3-methyl-2-butenoyl)-7-propionyl-10-deacetyl-cephalomannine

2'-tert-butyldimethylsilyl-2-debenzoyl-10-triethylsilyl-7-propionyl-cephalomannin e (20 mg,0.0206 mmol) was dissolved in 1 ml of toluene, and 3-methyl-2-butenoic acid (20.6 mg, 0.206 mmol), PP (1.8 mg, 0.0124 mmol) and DCC (42.5 mg, 0.206 mmol) were added. After reacting at 65 °C for 10 h, 0.1 ml methanol was added, then filtered. The solid was washed with ethyl acetate. The filtrates were combined and evaporated to dryness, followed by a silica gel column chromatography (petroleum ether : ethyl acetate = 8: 1). The resulting product was contaminated with impurities such as DCU, and said product was deprotected, without a further purification, as follows. The product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml). The aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane: ethyl acetate : acetone = 8: 3: 2) was carried out to yield the title product (7.5 mg, 45.2% of total yield over two steps). $^1H$ ($CDCl_3$, 300MHz): δ 7.29-7.42 (5H, m), 6.59 (1H, d, *J* = 8.7 Hz), 6.46 (1H, q, *J* = 6.9 Hz), 6.13 (1H, t, *J* = 8.9 Hz), 5.69 (1H, s), 5.58 (1H, dd, *J* = 1.8, 8.7 Hz), 5.39-5.45 (2H, overlapped), 5.25 (1H, s), 4.91 (1H, d, *J* = 7.8 Hz), 4.67 (1H, d, *J* = 1.8 Hz), 4.46 (1H, d, *J* = 8.1 Hz), 4.20 (1H, d, *J* = 8.1 Hz), 3.97 (1H, brs), 3.85 (1H, d, *J* = 6.3 Hz), 3.47 (1H, brs), 2.49 (1H, ddd, *J* = 7.3, 9.6, 14.5 Hz), 2.28 (1H, dd, *J* = 9.0, 15.3 Hz), 2.24 (2H, q, *J* = 7.2 Hz), 2.23 (3H, s), 2.20 (3H, s), 2.16 (1H, dd, *J* = 15.4, 9.0 Hz), 1.97 (3H, s), 1.91 (1H, m), 1.85 (3H, s), 1.79 (3H, s), 1.79 (3H, s), 1.76 (3H, d, *J* = 7.2 Hz), 1.20 (3H, s), 1.08 (3H, t, *J* = 8.1 Hz), 1.04 (3H, s). ESI-MS: m/z $[M+Na]^+$ 846.3.3, $[M+K]^+$862.3.

**Example 11: Preparation of 2-(3-methyl-3-butenoyl)-7-propionyl-10-deacetyl -cephalomannine**

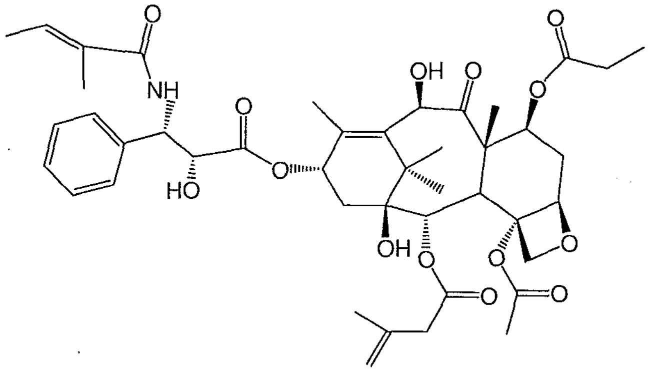

Steps 1-6: Being the same as the procedures described in Steps 1-6 of Example 10. It was obtained as a side product in Step 6 of Example 10, 27% yield.

$^1H$ ($CDCl_3$, 300MHz): δ 7.29-7.42 (5H, m), 6.59 (1H, d, *J* = 8.7 Hz), 6.46 (1H, q, *J* = 6.7 Hz), 6.11 (1H, t, *J* = 8.7 Hz), 5.58 (1H, d, *J* = 8.7 Hz), 5.41 and 5.40 (2H, overlapped), 5.24 (1H, s), 4.99 (1H, s), 4.92 (2H, brs), 4.67 (1H, d, *J* = 1.8 Hz), 4.51 (1H, d, *J* = 8.1 Hz), 4.23 (1H, d, *J* = 8.1 Hz), 3.95 (1H, brs), 3.84 (1H, d, *J* = 6.3 Hz), 3.48 (1H, brs), 3.11 (1H, d, *J* = 15.9 Hz), 3.03 (1H, d, *J* = 15.0 Hz), 2.49-2.53 (1H, m), 2.17-2.29 (3H, m), 2.22 (3H, s), 2.12 (1H, dd, *J* = 9.0, 15.4 Hz), 1.92 (1H, m), 1.87 (3H, s), 1.85 (3H, s), 1.79 (3H, s), 1.78 (3H, s), 1.76 (3H, d, *J* = 7.2 Hz), 1.19 (3H, s), 1.08 (3H, t, *J* =7.5 Hz), 1.01 (3H, s). ESI-MS: *m/z*, $[M+Na]^+$ 846.3.3, $[M+K]^+$ 862.3.

**Example 12: Preparation of 2-(3-methylbenzoyl)-7-propionyl-10-deacetyl -cephalomannine**

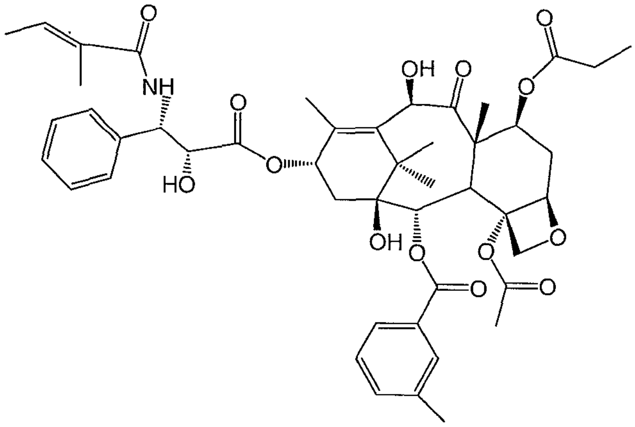

Steps 1-5: Being the same as the procedures described in Steps 1-5 of Example 7.

Step 6: 2-(3-methylbenzoyl)-7-propionyl-10-deacetyl-cephalomannine

2'-tert -butyldimethylsilyl-2-debenzoyl-10-triethylsilyl-7-propionyl-cephalomannin e (20.6 mg,0.0213 mmol) was dissolved in 1 ml of toluene, m-methylbenzoic acid (58 mg, 0.426 mmol), PP (4 mg, 0.027 mmol) and DCC (87.7 mg, 0.425 mmol) were added. After reacting at 65 °C for 10 h, 0.1 ml methanol was added, then filtered. The solid was washed with ethyl acetate. The filtrates were combined and evaporated to dryness, followed by a silica gel column chromatography (petroleum ether : ethyl acetate = 8: 1). The resulting product was contaminated with impurities such as DCU, and said product was deprotected, without a further purification, as follows. The product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml). The aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane : ethyl acetate : acetone = 8: 3: 2) was carried out to yield the title product (8.1 mg, 44.4% of total yield over two steps). $^1$H ($CDCl_3$, 300MHz): δ 7.89-7.93 (2H, m), 7.28-7.41 (7H, m), 6.62 (1H, d, *J* = 9.3 Hz), 6.44 (1H, q, *J* = 6.6 Hz), 6.13 (1H, t, *J* = 8.4 Hz), 5.66 and 5.62 (2H, overlapped), 5.45 (1H, dd, *J* = 7.2, 10.8 Hz), 5.28 (1H, s), 4.92 (1H, d, *J* = 9.6 Hz), 4.71 (1H, d, *J* = 1.8 Hz), 4.32 (1H, d, *J* = 8.4 Hz), 4.21 (1H, d, *J* = 7.5 Hz), 3.96 (1H, d, *J* = 6.6 Hz), 3.96 (1H, brs, overlapped), 3.57 (1H, brs), 2.48-2.58 (1H, m), 2.43 (3H, s), 2.36 (3H, s), 2.25 (2H, q, *J* = 7.5 Hz), 2.20-2.30 (2H, m, overlapped), 1.91 to 1.96 (1H, m), 1.85 (3H, s), 1.82 (6H, s), 1.72-1.74 (3H, d, *J* = 6.9 Hz), 1.21 (3H, s), 1.08 (3H, t, *J* = 7.5 Hz), 1.08 (3H, s). ESI-MS: m/z $[M+Na]^+$ 882.4.

**Example 13: Preparation of 2-(3-cyanobenzoyl)-7-propionyl-10-deacetyl -cephalomannine**

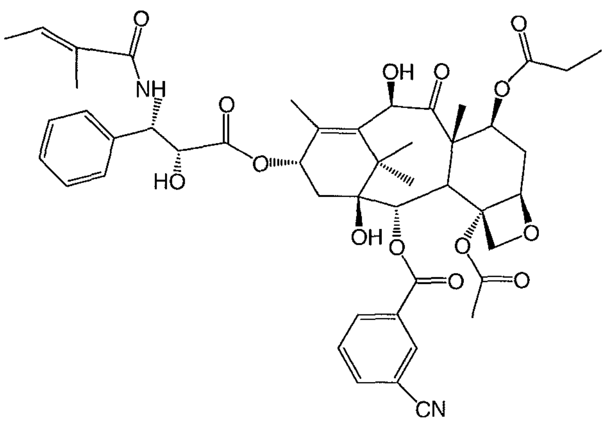

Steps 1-5: Being the same as the procedures described in Steps 1-5 of Example 7.

Step 6: 2-(3-cyanobenzoyl)-7-propionyl-10-deacetyl-cephalomannine

2'-tert-butyldimethylsilyl-2-debenzoyl-10-triethylsilyl-7-propionyl-10-deacetyl-ce phalomannine (12.3 mg, 0.0127 mmol) was dissolved in 1 ml of toluene, m-cyanobenzoic acid (18.6 mg, 0.127 mmol), PP (1.2 mg, 0.0081 mmol) and DCC (26.22 mg, 0.127 mmol) were added. After reacting at 65 °C for 10 h, 0.1 ml methanol was added, then filtered. The solid was washed with ethyl acetate. The filtrates were combined and evaporated to dryness, followed by a silica gel column chromatography (petroleum ether : ethyl acetate = 8: 1). The resulting product was contaminated with impurities such as DCU, and said product was deprotected, without a further purification, as follows. The product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml). The aqueous layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane: ethyl acetate : acetone = 8: 3: 2) was carried out to yield the title product (6 mg, 54.4% of total yield over two steps).
$^1H$ ($CDCl_3$, 300MHz): δ 8.43 (1H, s), 8.36 (1H, d, *J* = 7.5 Hz), 7.90 (1H, d, *J* = 7.8 Hz), 7.67 (1H, t, *J* = 7.2 Hz), 7.32-7.42 (5H, m), 6.53 (1H, d, *J* = 8.7 Hz), 6.50 (1H, q, *J* = 6.6 Hz), 6.16 (1H, t, *J* = 8.7 Hz), 5.65 and 5.63 (2H, overlapped), 5.47 (1H, dd, *J* = 4.8, 11 Hz), 5.31(1H, s), 4.93 (1H, d, *J* = 9.0 Hz), 4.71 (1H, d, *J* = 2.4 Hz), 4.25 (1H, d, *J* = 7.5 Hz), 4.19 (1H, d, *J* = 8.9 Hz), 4.00 (1H, d, *J* = 6.6 Hz), 2.46-2.58 (1H, m), 2.36 (3H, s), 2.26 (2H, q, *J* = 7.8 Hz), 2.22-2.29 (2H, m, overlapped), 1.92-1.97 (1H, m), 1.85 (6H, s), 1.79 (3H, s), 1.72 (3H, d, *J* = 6.9 Hz), 1.22 (3H, s), 1.09 (3H, t, *J* = 7.5 Hz), 1.08 (3H, s). ESI-MS: m/z $[M+Na]^+$ 893.4, $[M+K]^+$ 909.4.

**Example 14: Preparation of 2-(2-butenoyl)-7-propionyl-10-deacetyl -cephalomannine**

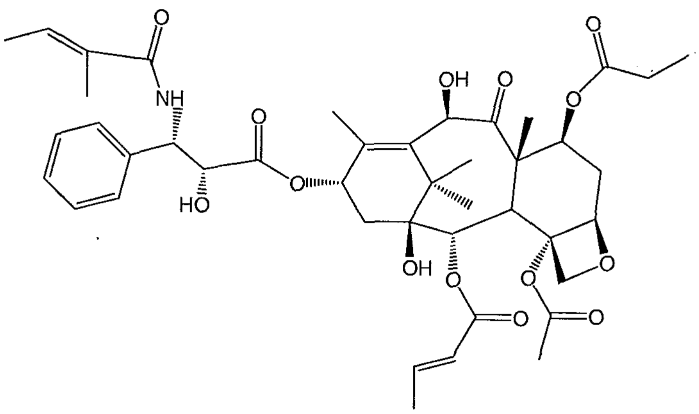

Steps 1-6 are similar to the procedures described in Steps 1-6 of Example 7, except that *m*-methoxybenzoic acid in Step 6 was substituted by 2-butenoic acid.

Steps 1-5: Being the same as the procedures described in Steps 1-6 of Example 7.

Step 6: 2-(2-butenoyl)-7-propionyl-10-deacetyl-cephalomannine

2'-tert-butyldimethylsilyl-2-debenzoyl-10-triethylsilyl-7-propionyl-cephalomannin e (37.9 mg,0.039 mmol) was dissolved in 1 ml of toluene, then 2-butenoic acid (33.57 mg, 0.39 mmol), PP (5.78 mg, 0.039 mmol) and DCC (80.5 mg, 0.39 mmol) were added. After reacting at 65 °C for 10 h, 0.1 ml methanol was added, then filtered. The solid was washed with ethyl acetate. The filtrates were combined and evaporated to dryness, followed by a silica gel column chromatography (petroleum ether : ethyl acetate = 8: 1). The resulting product was contaminated with impurities such as DCU, and said product was deprotected, without a further purification, as follows. The product was dissolved in 1.4 ml of acetonitrile, then pyridine (0.683 ml) and HF (0.375 ml) were added sequentially. After reacting at room temperature for 24 h, 30 ml of ethyl acetate was added, then washed with saturated aqueous $NaHCO_3$ (2 x 20 ml). The aqueous

layer was extracted with ethyl acetate (3 x 50 ml). The ethyl acetate layers were combined, and dried over anhydrous sodium sulfate. The ethyl acetate layer was evaporated to dryness. And a silica gel column chromatography (n-hexane : ethyl acetate : acetone = 8: 3: 2) was carried out to yield the title product (20.4 mg, 69% of total yield over two steps). $^1$H ($CDCl_3$, 300MHz): δ 7.32-7.40 (5H, m), 7.10 (1H, dq, *J* = 7.8, 15.1 Hz), 6.58 (1H, d, *J* = 9.3 Hz), 6.46 (1H, q, *J* = 6.9 Hz), 6.14 (1H, t, *J* = 8.4 Hz), 5.88 (1H, d, *J* = 15.1 Hz), 5.59 (1H, dd, *J* = 2.4, 9.3 Hz), 5.46 (1H, d, *J* = 6.6 Hz), 5.43 (1H, dd, *J* = 7.8, 10.7 Hz), 5.26 (1H, s), 4.92 (1H, dd, *J* = 2.2, 9.3 Hz), 4.68 (1H, d, *J* = 2.4 Hz), 4.44 (1H, d, *J* = 8.4 Hz), 4.20 (1H, d, *J* = 9.3 Hz), 3.96 (1H, brs), 3.87 (1H, d, *J* = 6.9 Hz), 3.44 (1H, brs),

2.46-2.53 (1H, m), 2.26 (3H, s), 2.22-2.31 (2H, m, overlapped), 1.96 (3H, d, *J* = 6.9 Hz), 1.92 (1H, m, overlapped), 1.88 (3H, s), 1.80 (3H, s), 1.79 (3H, s), 1.76 (3H, d, *J* = 6.6 Hz), 1.20 (3H, s), 1.08 (3H, t, *J* = 6.9 Hz), 1.04 (3H, s). ESI-MS: *m*/*z* $[M+Na]^+$ 832.3.

**Pharmacological experiments:**

**Experiment 1 Cytotoxicity screening assay at cell level of compounds of this invention**

Tumor cells at log phase were incubated on 96-well plates at a density of 1 ~ $1.2 \times 10^4$ cells/ml, 100μl/well. 24h later, treatment cells were exposed to the tested agents at different concentrations. Each tested agents had 4 ~ 5 doses, and each dose had at least 3 parallel wells. Control cells were exposed to solvents in the same volume with treat agents. Cells were cultured at 37°C in a cell incubator with 5% $CO_2$ and 95% air atmosphere. After 4 days of cultrue, the incubation medium was removed, then 200μl 0.2% MTT (prepared by RPMI 1640) was added to each well and the plates were incubated at 37°C for 4 hours. The supernatant was discarede and dissolved the resulting formazen product with 200μl DMSO in each well. The absorbance at a reference wavelenth of 450nm and a test wavelength of 570nm was read on a microplate reader after tenderly shaking. Calculated drug inhibitory rate using the following equation and $IC_{50}$, taking solvent exposure tumor cells as control cells.

$$\text{inhibitory rate} = \frac{\text{average OD value of control cells} - \text{the average OD value of treated cells}}{\text{average OD value of control cells}} \times 100\%$$

( results are listed in Table 1 )

**Table 1.In vitro cytotoxicity results of some compounds of the invention**

| Sample | $IC_{50}$(M) | | | |
|---|---|---|---|---|
| | KB | KB/V | A549 | A549/Taxol |
| Example 1 | $3.37 \times 10^{-7}$ | $>1 \times 10^{-6}$ | $>1 \times 10^{-6}$ | $>1 \times 10^{-6}$ |
| Example 2 | $1.03 \times 10^{-9}$ | $1.53 \times 10^{-7}$ | $8.79 \times 10^{-8}$ | $>1 \times 10^{-6}$ |
| Example 3 | $1.38 \times 10^{-7}$ | $8.02 \times 10^{-7}$ | $7.04 \times 10^{-8}$ | $>1 \times 10^{-6}$ |
| Example 4 | $9.74 \times 10^{-9}$ | $4.85 \times 10^{-7}$ | $2.01 \times 10^{-7}$ | $4.21 \times 10^{-7}$ |
| Example 5 | $5.90 \times 10^{-10}$ | $2.23 \times 10^{-7}$ | $>1 \times 10^{-6}$ | $>1 \times 10^{-6}$ |
| Example 6 | $1.44 \times 10^{-7}$ | $8.94 \times 10^{-7}$ | $2.10 \times 10^{-7}$ | $>1 \times 10^{-6}$ |
| Example 7 | $8.78 \times 10^{-9}$ | $2.79 \times 10^{-7}$ | $2.51 \times 10^{-7}$ | $>1 \times 10^{-6}$ |
| Example 8 | $2.39 \times 10^{-9}$ | $2.73 \times 10^{-7}$ | $9.36 \times 10^{-9}$ | $>1 \times 10^{-6}$ |
| taxol | $8.66 \times 10^{-9}$ | $9.59 \times 10^{-7}$ | $2.32 \times 10^{-7}$ | $>1 \times 10^{-6}$ |
| cephalomannine | $5.18 \times 10^{-9}$ | $1.00 \times 10^{-6}$ | $1.33 \times 10^{-7}$ | $>1 \times 10^{-6}$ |
| docetaxel | $9.33 \times 10^{-8}$ | $8.90 \times 10^{-7}$ | $2.19 \times 10^{-7}$ | $>1 \times 10^{-6}$ |

KB: the human oral epithelium cancer cell strain
KB/V:the vincristine -resistant substrain of KB
A549: the human lung adenocarcinoma cell strain
A549/Taxol: the taxol-resistant substrain of A549

**Table 2. Cytotoxicity of the compound of example 8 on several tumor cell strains by MTT assay**

| Cell strains | $IC_{50}$(mol/L) | |
|---|---|---|
| | Taxol | Lx2-32c |
| HCT-8 | $7.3 \times 10^{-10}$ | $3.2 \times 10^{-10}$ |
| Bel-7402 | $1.3 \times 10^{-8}$ | $8.7 \times 10^{-9}$ |
| A2780 | $1.3 \times 10^{-9}$ | $3.6 \times 10^{-9}$ |
| A549 | $5.2 \times 10^{-9}$ | $9.4 \times 10^{-10}$ |
| A549/T | $2.6 \times 10^{-8}$ | $1.4 \times 10^{-9}$ |
| MCF-7 | $4.1 \times 10^{-10}$ | $6.8 \times 10^{-10}$ |
| MCF-7/D | $3.3 \times 10^{-7}$ | $2.7 \times 10^{-7}$ |
| BGC-823 | $6.3 \times 10^{-9}$ | $1.3 \times 10^{-9}$ |
| BGC-803 | $2.5 \times 10^{-9}$ | $2.4 \times 10^{-9}$ |
| KB | $1.8 \times 10^{-9}$ | $4.9 \times 10^{-10}$ |

A549/T: the taxol-resistant substrain of the human lung adenocarcinoma cell strain A549 ;
MCF-7/D: adriamycin-resistant subline of the human mammary adenocarcinoma cell strain MCF-7

**Experiment 2 In vivo activity experiments of the compounds of the invention**

**1 Tested agents:**

Name: Example 8, taxol

Preparation: both example 8 and taxol are active pharmaceutical ingredients,white powder.

Compound method: Taxol and example 8 were both prepared to the required concentration using solvent for injection which was provided by Peking union pharmaceutical factory.

**2 Experimental animals :**

Source: Animal breeding field of the institute of Laboratory Animal Research, Chinese Academy of Medical Sciences

Genera: BALB/c (nu-nu) nude mice
Certification Number: Beijing animal permission No. 017
Weight: 19-22g
Gender: ♀
Animal numbers of each group:6-8 each group
Breeding environment: SPF

**3 Methods:**

After one-week accommodation, animals were given subcutaneous implantation of the human lung adenocarcinoma A549 or the human ovarian cancer A2780 or the human gastric carcinoma BGC-823 cells. Animals were grouped randomly (d0) when the tumors reached 100-300mm$^3$. Daily i.p. administration of Taxol (10mg/kg) and example 8 (5mg/kg) on the human lung adenocarcinoma A549-nude mice- xenograft tumor from d0 to d4 for totally 5 times; Taxol (30mg/kg) on the human ovarian cancer A2780-nude mice- xenograft tumor from d0 to d3 for totally 4 times and on the human gastric carcinoma BGC-823 nude mice- xenograft tumor from d0 to d2 for totally 3 times; example 8 (5mg/kg, 15mg/kg, 30mg/kg) on both the human ovarian cancer A2780 and BGC-823 nude mice- xenograft tumor from d0 to d3 for totally 4 times was initiated. Animal weights and tumor mesuraments were taken every 2 weeks and recorded. At the end of the study(d30 or d35), nude mice were euthanized and weighed. After the animals were sacrificed, tumor tissue was cut and weighed. Tumor volume was determined using the following equation:

$$V = 1/2 \times a \times b^2$$

wherein a represents length while b represents width

Growth inhibitory effects of the compounds of the invention on the human ovarian cancer A2780-nude mice- xenograft tumor are listed in Figure 3.

Growth inhibitory effects of the compounds of the invention on the human gastric carcinoma BGC-823-nude mice-xenograft tumor are listed in Figure 4.

Growth inhibitory effects of the compounds of the invention on Lewis lung cancer in mice are listed in Figure 5.

Growth inhibitory effects of the compounds of the invention on the human lung adenocarcinoma A549-nude mice-xenograft tumor are listed in Figure 6.

**Table 3.Growth inhibitory activity of the compounds of the invention on A2780-nude mice- xenograft tumor**

| Group | Dosage ( mg/kg) | Animals | | body weight(g) | | Tv x±SD | | RTV | T/C (%) |
|---|---|---|---|---|---|---|---|---|---|
| | | d0 | dn | initial | end | d0 | dn | | |
| control | | 7 | 7 | 22.0 | 25.1 | 149.9±59.0 | 1447.4±941.2 | 9.7 | |
| taxol | 30x4 | 7 | 6 | 22.7 | 23.3 | 137.9±21.0 | 604.8±525.0* | 4.4 | 45.4 |
| | 7.5x4 | 7 | 6 | 22.4 | 25.5 | 127.6±65.9 | 989.3±658.9 | 7.8 | 80.3 |
| Example 8 | 15x4 | 7 | 5 | 22.0 | 25.2 | 130.2±36.0 | 855.6±500.3 | 6.6 | 68.1 |
| | 30x4 | 7 | 6 | 22.0 | 22.3 | 157.1±56.2 | 529.6±454.8* | 3.4 | 34.9 |

Note: d0 : time of the first administration since raising in separated cages; dn : 30 days after the first administration. *P0<0.05 vs control

**Table 4. Growth inhibitory activity of the compounds of the invention on BGC-823-nude mice- xenograft tumor**

| Group | Dosage (mg/kg) | Animals d0/dn | Body weight(g) | | Tv x±SD | | RTV | T/C (%) |
|---|---|---|---|---|---|---|---|---|
| | | | initial | end | d0 | d | | |
| Control | | 7/7 | 26.0 | 27.7 | 136.3±33.5 | 3290.3±709.8 | 27.33 | |
| taxol | 30x3 | 7/4 | 25.2 | 23.9 | 98.8±47.35 | 104.9±144.9** | 0.66 | 2.41 |
| | 7.5x4 | 7/5 | 24.7 | 26.1 | 101.1±17.4 | 2057.5±1756.4 | 17.45 | 63.84 |
| Example 8 | 15x4 | 7/4 | 25.2 | 23.8 | 104.4±16 | 1156.6±403.9* | 9.27 | 33.90 |
| | 30x4 | 7/5 | 25.0 | 23.9 | 99.7±34.3 | 177.1±199.0** | 0.47 | 4.57 |

Note: d0 : time of the first administration since raising in separated cages; dn 30 days after the first administration. *P0<0.05 vs control

**Table 5.The inhibitory activity of Lx2-32c on mice Lewis lung cancer**

| Group | Dosage (mg/kg) | Body weight(g) | | *Tumor weight(g)* | | *Inhibition* (%) |
|---|---|---|---|---|---|---|
| | | initial | end | Average | SD | |
| Control | | 19.6 | 24.0 | 2.87 | 1.13 | |
| taxol | 10x6 | 19.7 | 19.4 | 1.96 | 0.81 | 31.64 |
| Lx2-32c | 2.5x8 | 20.0 | 22.0 | 2.07 | 0.22 | 27.77 |
| | 5x8 | 20.0 | 20.4 | 1.96 | 0.68 | 32.46 |
| | 10x8 | 19.5 | 16.8 | 0.69 | 0.33 | 76.08 |

**Table 6.Growth inhibitory activity of compounds of this invention on A549- nude mice- xenograft tumor**

| Group | Dosage (mg/kg) | Animals d0/dn | Body weight(g) | | Tv x+SD | | RTV | T/C (%) |
|---|---|---|---|---|---|---|---|---|
| | | | initial | end | initial | end | | |
| control | | 7/7 | 22.1 | 27.6 | 411±136 | 4862±1861 | 11.82 | |
| taxol | 30x3 | 8/6 | 22.0 | 24.3 | 468±135 | 2152±474 | 4.6 | 38.9 |
| | 7.5x3 | 7/6 | 21.6 | 21.8 | 431±94 | 3090±900 | 7.2 | 60.7 |
| Lx2-32c | 15x3 | 8/7 | 22.4 | 25.1 | 432±115 | 2897±950 | 6.7 | 56.7 |
| | 30x3 | 8/6 | 21.8 | 23.2 | 415±85 | 1646±450 | 4.0 | 33.56 |

**4. Conclusion**

Taxol can inhibit the growth of the human lung adenocarcinoma cell line A549, the human ovarian cancer cell strain A2780, Lewis lung cancer and the human gastric carcinoma cell strain BGC-823 nude mice- xenograft tumor. The compound of example 8 has a stronger effect on the human lung adenocarcinoma cell strain A549, the human ovarian cancer cell strain A2780, Lewis lung cancer nude mice- xenograft tumor than taxol. The inhibitory effect on the human gastric carcinoma cell strain BGC-823 nude mice- xenograft tumor of the compound of example 8 is equal to taxol.

**Claims**

1. A compound selected from cephalomannine derivatives of general formula (I),

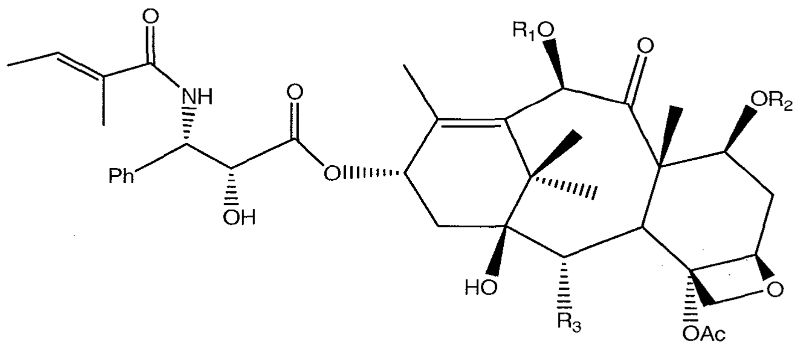

(I)

**characterized in that**
$R_1$ is selected from the group consisting of hydrogen, TMS, TES, TBS and $-COX_1$, $X_1$ is selected from $C_1$-$_5$ alkyl;
$R_2$ is selected from the group consisting of hydrogen, substituted or unsubstituted straight or branched $C_1$-$_{15}$ alkyl, $C_2$-$_{15}$ alkenyl, $C_2$-$_{15}$ alkynyl, un-, mono- or multi-substituted aryl and heteroaryl, $-COX_2$; $-COX_3-COOX_4$; $-COX_3-CONX_4X_5$;
$R_3$ is selected from the group consisting of hydrogen, substituted or unsubstituted straight or branched $C_1$-$_{15}$ alkyl, $C_2$-$_{15}$ alkenyl, $C_2$-$_{15}$ alkynyl, un-, mono- or multi-substituted aryl and heteroaryl, $-OX_6$; $-SX_6$; $-NHX_6$; $-OCOX_6$; and,
$X_2$, $X_3$, $X_4$, $X_5$ and $X_6$ are independently selected from the group consisting of hydrogen; substituted or unsubstituted straight or branched $C_1$-$_{15}$ alkyl, $C_2$-$_{15}$ alkenyl, $C_2$-$_{15}$ alkynyl, un-, mono- or multi-substituted aryl and heteroaryl;
the substituents for said alkyl are selected from the group consisting of hydroxy, amino, carboxyl, carbonyl, $C_1$-$_5$ alkoxy, halo, $C_1$-$_5$ alkoxycarbonyl, N-$C_1$-$_5$ alkylcarbamoyl, cyano, nitro;
the substituents for said aryl and heteroaryl are selected from the group consisting of hydroxy, hydroxymethyl, halo, $C_1$-$_5$ alkyl, $C_1$-$_5$ alkoxy, $C_1$-$_5$ alkenyl, acyl, acyloxy, nitro, amino, amido, cyano, azido.

2. The compound according to claim 1, **characterized in that**
the $C_{1-15}$ alkenyl is selected from the group consisting of vinyl, propenyl, isopropenyl, butenyl, isobutenyl, hexenyl;
the $C_{1-15}$ alkynyl is selected from the group consisting of ethynyl, propinyl, isopropinyl, butynyl, isobutynyl, hexynyl;
the aryl is selected from the group consisting of phenyl, naphthyl, biphenyl;

the heteroaryl is selected from the group consisting of furyl, thienyl, pyridyl, benzofuryl, bipyridyl;
the halo is selected from the group consisting of F, Cl, Br, and I.

3. The compound according to claim 2, **characterized in that** $R_1$ and $R_2$ are independently selected from the group consisting of hydrogen, formyl, acetyl, propionyl, butyryl.

4. The compound according to claim 3, **characterized in that** said compound is selected from the group consisting of:

   10-deacetyl-cephalomannine
   2-(3-azidobenzoyl)-cephalomannine
   2-(3-methoxybenzoyl)-cephalomannine
   2-(3-azidobenzoyl)-10-propionyl-cephalomannine 2-(3-methoxybenzoyl)-10-propionyl-cephalomannine
   10-propionyl-cephalomannine 2-(3-methoxybenzoyl)-7-propionyl-cephalomannine
   2-(3-azidobenzoyl)-7-propionyl-cephalomannine 2-(3-chlorobenzoyl)-7-propionyl-cephalomannine 2-(3-methylbenzoyl)-7-propionyl-cephalomannine 2-(3-cyanobenzoyl)-7-propionyl-cephalomannine 2-(3-methyl-2-butenoyl)-7-propionyl-cephalomannine 2-(3-methyl-3-butenoyl)-7-propionyl-cephalomannine
   2-(2-butenoyl)-7-propionyl-cephalomannine

5. A process for preparation of the compound according to claims 1 to 4, **characterized in that**

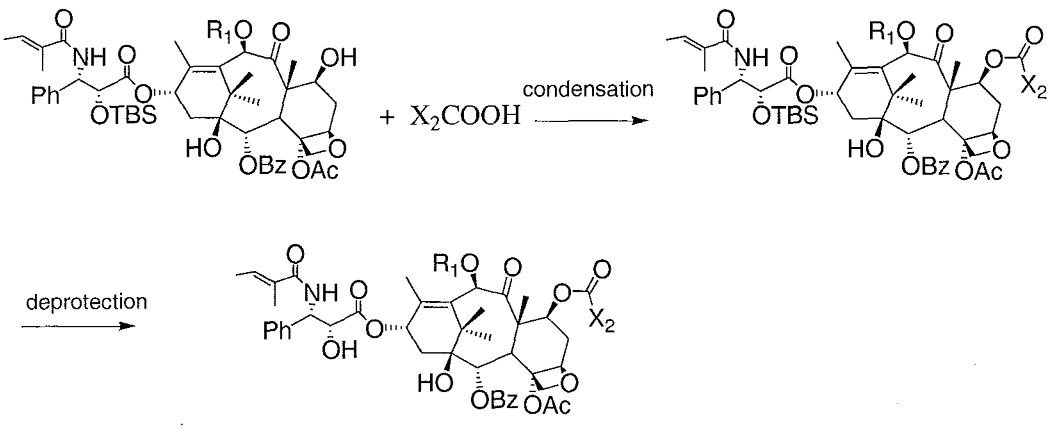


IIa

the hydroxy at C-7 position of the cephalomannine modified or unmodified at C-10 position as starting material was condensed with a corresponding acid or acyl chloride to produce the compound of formula IIa;
or

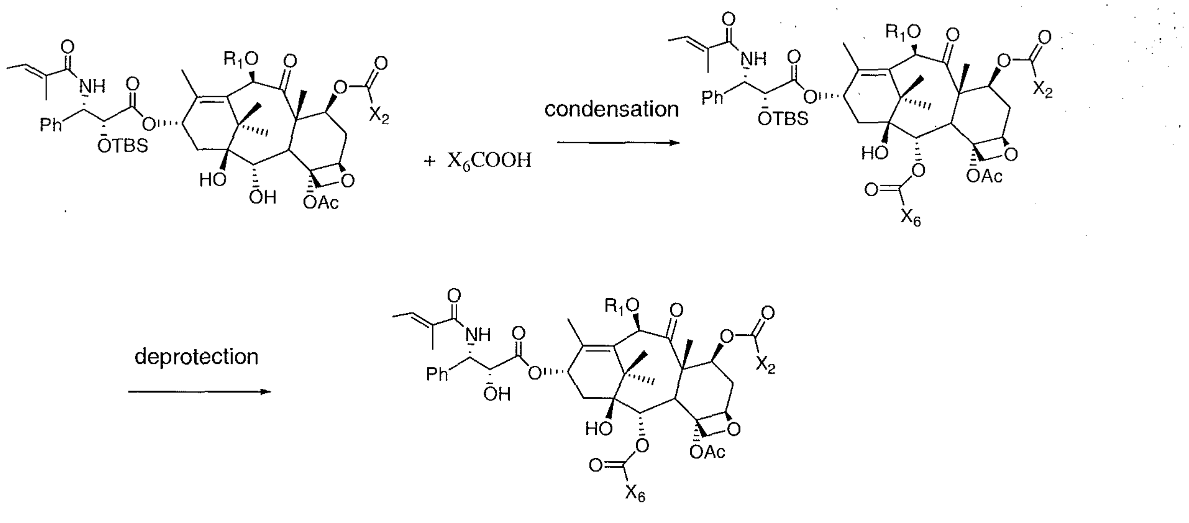


IIb

the cephalomannine modified at both C-10 and C-7 positions, after the removal of benzoyl at 2-position, were condensed with a corresponding acid or acyl chloride to produce the compound of formula IIb.

6. The process according to claim 5, **characterized in that** the condensation agents used in the acylation reaction include 1,3-dicyclohexylcarbodiimide, dipyridyl carbonate, 1,3-diisopropyl carbodiimide, 1-(3-dimethylaminopropyl)-3-ethylcarbodiimide.

7. The process according to claim 5, **characterized in that** the catalysts used in the acylation reaction include tertiary amines, pyridine, 4-dimethylaminopyridine and 4-pyrrolylpyridine.

8. The process according to claim 5, **characterized in that** the organic solvents used in the acylation reaction include dimethylsulfoxide, toluene, methylene chloride, ethylene glycol dimethyl ether, 1,2-dichloroethane, tetrahydrofuran and N,N-dimethylformamide.

9. A pharmaceutical composition, **characterized in that** it comprises at least one compound according to claims 1-4 as active ingredient and pharmaceutically acceptable carriers.

10. Use of the compound according to any one of claims 1-4 in the manufacture of a medicament for the treatment of tumors.

11. The use according to claim 11, **characterized in that** the tumors include multidrug resistant human pulmonary adenocarcinoma, multidrug resistant ovarian cancer, multidrug resistant human gastric cancer, sensitive human pulmonary adenocarcinoma, sensitive ovarian cancer, sensitive human gastric cancer.

# INTERNATIONAL SEARCH REPORT

International application No.
PCT/CN2007/003235

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC: C07D305/-,A61K31/-,A61P

Documentation searched other than minimum documentation to the extent that such documents are included in the fields searched

Electronic data base consulted during the international search (name of data base and, where practicable, search terms used)

WPI, EPODOC, PAJ, CNKI, CNPAT, CA, STN: Cephalomannine ; Taxol B ; Cephalmannine ; cephalomannie ; cephalommanine

C. DOCUMENTS CONSIDERED TO BE RELEVANT

| Category* | Citation of document, with indication, where appropriate, of the relevant passages | Relevant to claim No. |
|---|---|---|
| X | Eun Joo Roh et al., "Structure-Activity Relationship Study at the 3'-N-Position of Paclitaxel : Synthesis and Biological Evaluation of 3'-N-Acyl-Paclitaxel Analogues", Bioorganic &Medicinal Chemistry ,2002(10), p3145-3151, compounds 1g,6g | 1-11 |
| X | Neal F. Magri et al., "Modified Taxols. 3.Preparation and Acylation of Baccatin III", J. Org. Chem.,1986(51), p3239-3242, compounds 1b,3b | 1-8 |
| X | US2005/0240036A1 (PHYTOGEN LIFE SCIENCES INC.) 27 Oct. 2005(27.10.2005),page 2, compound (6) | 1-3 |
| X | CN1234700C (FUDAN UNIVERSITY) ,04 Jan. 2006 (04.01.2006) , Page 1, compound II | 1-3 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 15 Aug. 2008 (15.08.2008) | **04 Sep. 2008 (04.09.2008)** |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>LIU Wenxia<br>Telephone No. (86-10)62413682 |

Form PCT/ISA/210 (second sheet) (April 2007)

## INTERNATIONAL SEARCH REPORT

| International application No. |
|---|
| PCT/CN2007/003235 |

C (Continuation). DOCUMENTS CONSIDERED TO BE RELEVANT

| Category* | Citation of document, with indication, where appropriate, of the relevant passages | Relevant to claim No. |
|---|---|---|
| E | CN101074218A（INSTITUTE OF MATERIA MEDICA,CHINESE ACADEMY OF MEDICAL SCIENCES ET AL. ）,21 Nov. 2007（21.11.2007）,claims 1-9 | 1-11 |

Form PCT/ISA/210 (continuation of second sheet ) (April 2007)

**INTERNATIONAL SEARCH REPORT**
Information on patent family members

International application No.
PCT/CN2007/003235

| Patent Documents referred in the Report | Publication Date | Patent Family | Publication Date |
|---|---|---|---|
| US2005/0240036A1 | 27-10-2005 | WO2005105767A1 | 10-11-2005 |
| CN1234700C | 04-01-2006 | CN1523023A | 25-08-2004 |
| CN101074218A | 21-11-2007 | none | |

Form PCT/ISA/210 (patent family annex) (April 2007)

**INTERNATIONAL SEARCH REPORT**

International application No.
PCT/CN2007/003235

continued to page 2 **A. CLASSIFICATION OF SUBJECT MATTER**

| | |
|---|---|
| C07D305/14 | (2006.01) i |
| A61K31/337 | (2006.01) i |
| A61P35/00 | (2006.01) i |

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 4814470 A, Colin

### Non-patent literature cited in the description

- **Schiff, P. B. ; Fant, J. ; Horwitz S. B.** *Nature,* 1979, vol. 277, 665